# EUROPEAN PATENT SPECIFICATION

(11) **EP 4 006 046 B1**
(45) Date of publication and mention of the grant of the patent: **19.03.2025**
(21) Application number: 20846188.9
(22) Date of filing: 30.07.2020
(51) Int. Cl.: C07H 15/256, C07H 1/00, C07H 1/08, A23L 27/30

(54) **NOVEL STEVIOL GLYCOSIDE, METHOD FOR PRODUCING SAME, AND SWEETENER COMPOSITION CONTAINING SAME**
NEUARTIGES STEVIOLGLYKOSID, VERFAHREN ZUR HERSTELLUNG DAVON UND SÜSSSTOFFZUSAMMENSETZUNG DAMIT
NOUVEAU GLYCOSIDE DE STÉVIOL, SON PROCÉDÉ DE PRODUCTION ET COMPOSITION D'ÉDULCORANT LE CONTENANT

(30) Priority: 31.07.2019 JP 2019141627
(43) Date of publication of application: 01.06.2022
(73) Proprietor: Suntory Holdings Limited, Osaka 530-8203 (JP)
(72) Inventor: URAI Soichiro, Kawasaki-shi, Kanagawa 211-0067 (JP); IWAKI Kazunari, Kawasaki-shi, Kanagawa 211-0067 (JP); MIYAGAWA Katsuro, Soraku-gun, Kyoto 619-0284 (JP); HIRAI Tadayoshi, Soraku-gun, Kyoto 619-0284 (JP); NAGAO Koji, Kawasaki-shi, Kanagawa 211-0067 (JP); YOKOO Yoshiaki, Kawasaki-shi, Kanagawa 211-0067 (JP); WATANABE Takehiro, Soraku-gun, Kyoto 619-0284 (JP); FUJIKAWA Kohki, Soraku-gun, Kyoto 619-0284 (JP)
(74) Representative: Mewburn Ellis LLP
(86) International application number: PCT/JP2020/029271
(87) International publication number: WO 2021/020516

(56) References cited:
- WO-A1-2018/090020
- WO-A1-2018/181515
- PERERA, WILMER H. ET AL.: "Assignment of sugar arrangement in branched steviol glycosides using electrospray ionization quadrupole time-of-flight tandem mass spectrometry", RAPID COMMUNICATIONS IN MASS SPECTROMETRY, vol. 31, 2017, pages 315 - 324, XP055736572, ISSN: 1097-0231, DOI: 10.1002/rcm.7784
- PERERA WILMER H., GHIVIRIGA ION, RODENBURG DOUGLAS L., CARVALHO RAQUEL, ALVES KAMILLA, MCCHESNEY JAMES D.: "Development of a high- performance liquid chromatography procedure to identify known and detect novel C-13 oligosaccharide moieties in diterpene glycosides from Stevia rebaudiana (Bertoni) Bertoni (Asteraceae): Structure elucidation of rebaudiosides V and W", JOURNAL OF SEPARATION SCIENCE, vol. 40, 2017, pages 3771 - 3781, XP055789748, ISSN: 1615-9314
- FERERA, WILMER H. ET AL.: "Rebaudiosides T and U, minor C-19 xylopyranosyl and arabinopyranosyl steviol glycoside derivatives from Stevia rebaudiana (Bertoni) Bertoni", PHYTOCHEMISTRY, vol. 135, 2017, pages 106 - 114, XP029898124, ISSN: 0031-9422, DOI: 10.1016/j.phytochem.2016.12.001

## Description

### Technical Field

The present invention relates to a novel steviol glycoside, a method for producing the same, and a sweetener composition comprising the same. Furthermore, the present invention also relates to a food or beverage, a plant, an extract thereof and a flavor controlling agent comprising the novel steviol glycoside.

### Background Art

A leaf of *Stevia rebaudiana* contains a secondary metabolite called steviol which is one kind of diterpenoids, where the steviol glycosides provide sweetness that is nearly 300 times the sweetness of sugar and is therefore utilized as a calorieless sweetener in the food industry. The demand for calorieless sweeteners is growing day by day as obesity has become a serious social problem worldwide and also for the sake of health promotion and reduction in the medical expenditure. Currently, aspartame and acesulfame potassium, i.e., artificially synthesized amino acid derivatives, are utilized as artificial sweeteners, but natural calorieless sweeteners like the steviol glycosides are expected to be safer and more likely to gain public acceptance.

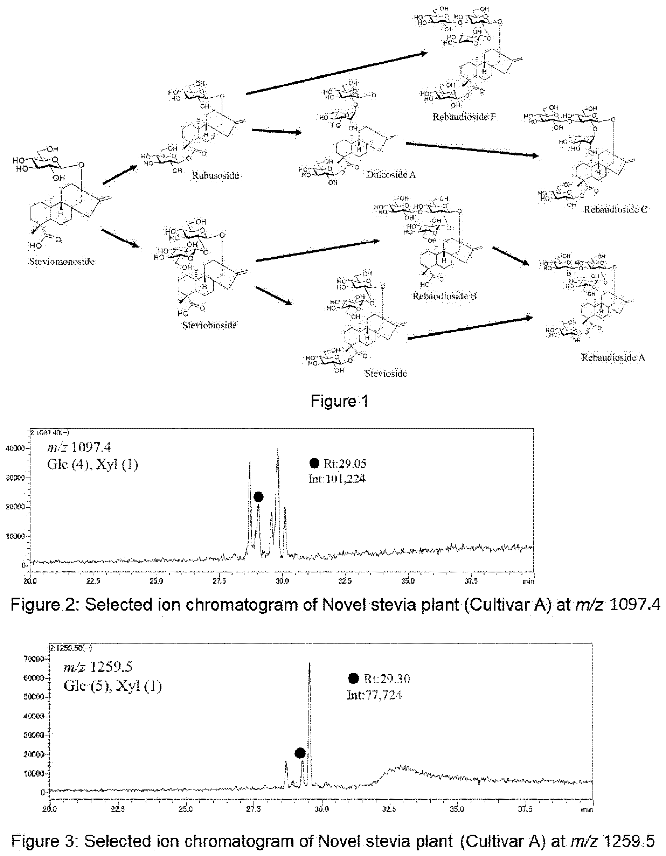

The major steviol glycosides from stevia are ultimately glycosylated to a glycoside called rebaudioside A (Reb.A) that has four sugar moieties (Figure 1). Stevioside, namely, a tri-glycosylated steviol glycoside which is a precursor of Reb.A, is the most abundant glycoside. These two glycosides are the main substances responsible for the sweetness of stevia. Stevioside accounts for the largest content in a stevia leaf and is known to provide sweetness that is about 250-300 times the sweetness of sugar. Reb.A is a tetra-glycosylated steviol glycoside that has strong sweetness (350-450 times sugar) with good taste quality. They have been drawing attention as calorieless sweeteners. Besides them, existence of glycosides that are considered to be reaction intermediates and analogs having different types of sugar moieties are known. For example, while all of the four glycoside sugar moieties of Reb.A are glucose, rebaudioside C (Reb.C) is known to have rhamnose instead of glucose attached to C-2 of glucose at C-13, and rebaudioside F (Reb.F) is known to have xylose attached at the same position.

To date, attempts have been made to obtain a stevia plant having a higher Reb.A content than wild-type stevia plants by variety improvement since taste quality of Reb.A, in which all of the four glycoside sugar moieties are glucose, is good (for example, Patent literature 1). In addition, an attempt has also been made to obtain a novel steviol glycoside by decomposing a known steviol glycoside such as rebaudioside M (Reb.M (also referred to as Reb.X)) that has good taste quality with an acid (for example, Patent literature 2). Patent literature 3 describes a purified composite of steviol glycoside molecules for imparting tast, flavour and flavour modifying properties to consumable products. Patent literature 4 describes the isolation of a novel steviol glycoside from a species containing a large amount of Reb. C that is capable of influencing the quality of taste.

### Citation List

### Patent Literature

[Patent Literature 1] Japanese Patent No. 3436317
[Patent Literature 2] International Patent Application Publication WO2014/146135
[Patent Literature 3] International Patent Application Publication WO2018/090020
[Patent Literature 4] International Patent Application Publication WO2018/181515

### Summary of Invention

### Problem to be Solved by the Invention

Since the sweetness level and the taste quality of steviol glycosides may vary greatly depending on the difference in the number and the type of the sugar moiety that attaches to the diterpene structure serving as a backbone, there has been a need for a novel steviol glycoside.

### Means for Solving the Problems

The present invention provides a novel steviol glycoside comprising xylose, and a sweetener composition, a food or beverage comprising the same as shown below.

In one aspect, there is provided a compound represented by Formula (3):

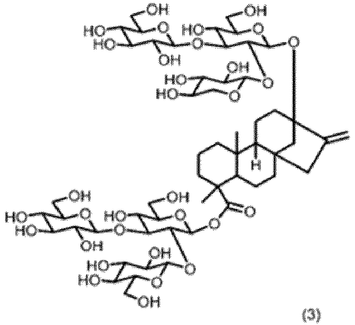

In some embodiments, the compound, or a salt or a hydrate thereof, is represented by Formula (5) below:

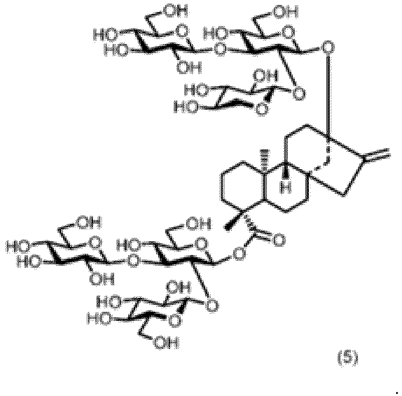

In a second aspect, there is provided a food or beverage comprising the compound of the first aspect.

In some embodiments, the content of the compound of the first aspect is 1-600 mass ppm. In a third aspect, there is provided a sweetener composition comprising the compound of the first aspect.

In some embdoiments, the content of the compound of the first aspect is 1-99 wt%.

In some embodiments, the sweetener composition further comprises one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside.

In a fourth aspect, there is provided a food or beverage comprising the sweetener composition of the third aspect.

In some embodiments, the food or beverage is a beverage.

In a fifth aspect, there is provided the use of the compound of the first aspect as a sweetener.

### Advantageous Effects of Invention

The present invention can provide a novel steviol glycoside comprising xylose, a method for producing the same, and a sweetener composition, a food or beverage, a plant, an extract thereof and a flavor controlling agent comprising the novel steviol glycoside. A steviol glycoside in a preferable aspect of the present invention has excellent taste quality and a high sweetness level. A sweetener composition in another aspect of the present invention has excellent taste quality.

### Brief Description of Drawings

Figure 1 is a diagram showing structures and names of steviol glycosides.
Figure 2 is a diagram showing a selected ion chromatogram of Cultivar A at m/z of 1097.4.
Figure 3 is a diagram showing a selected ion chromatogram of Cultivar A at m/z of 1259.5.
Figure 4 is a diagram showing MS/MS and MS³-fragmented mass spectra of Novel steviol glycoside 1E.
Figure 5 is a diagram showing MS/MS and MS³-fragmented mass spectra of Novel steviol glycoside 2E.

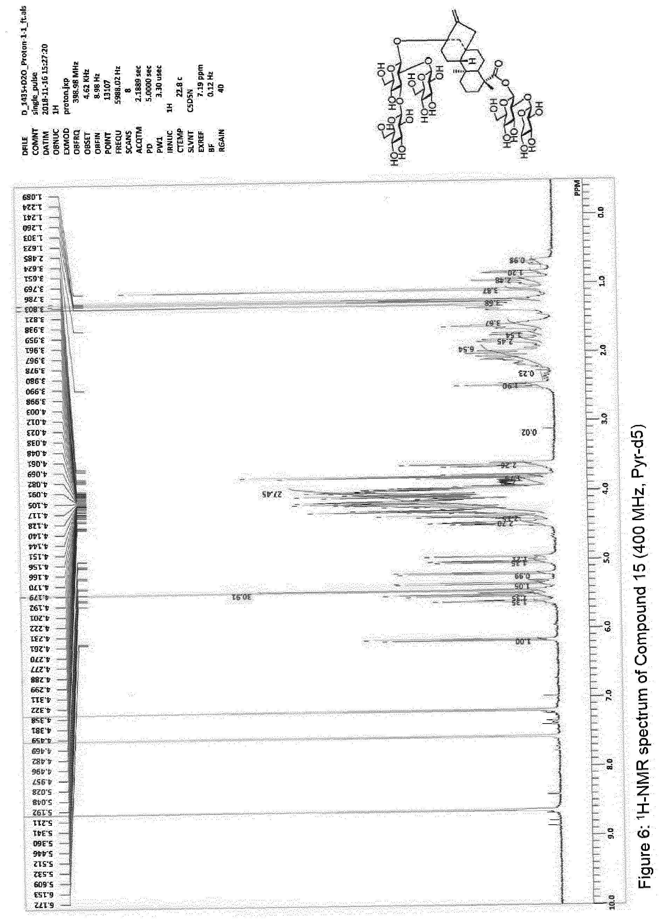

Figure 6 is a diagram showing a ¹H-NMR spectrum of Reference Compound 15 (400 MHz, Pyr-d5).

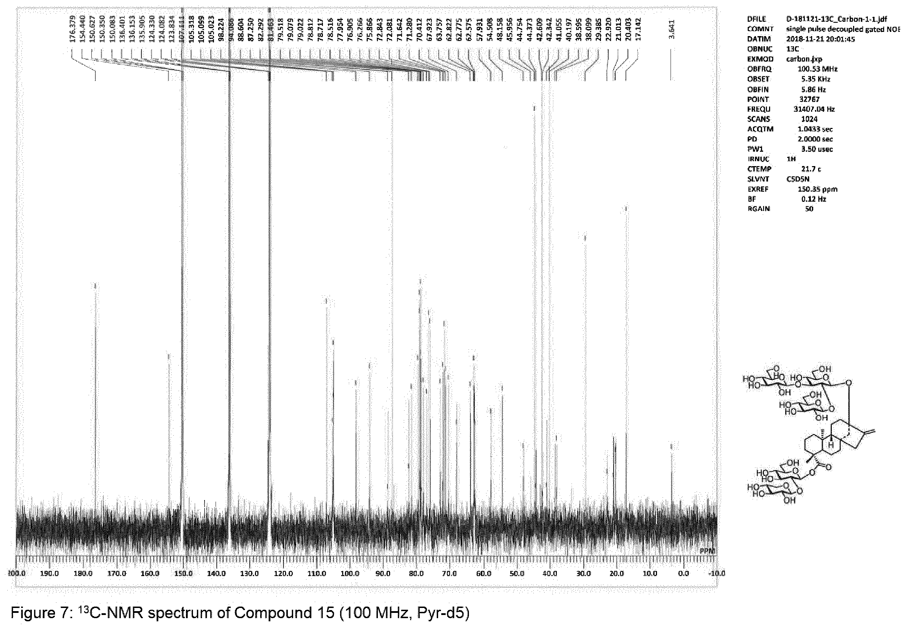

Figure 7 is a diagram showing a ¹³C-NMR spectrum of Reference Compound 15 (100 MHz, Pyr-d5).

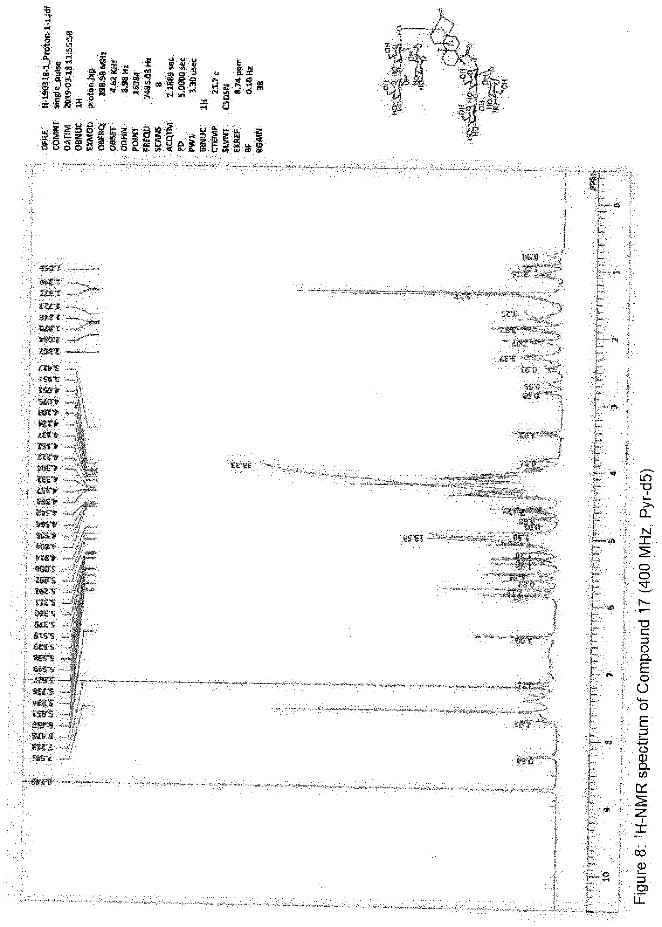

Figure 8 is a diagram showing a ¹H-NMR spectrum of Compound 17 (400 MHz, Pyr-d5).

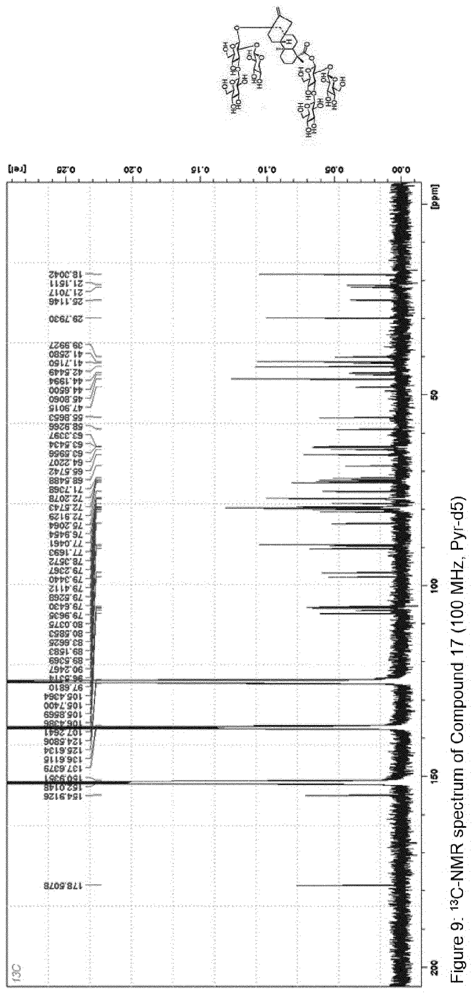

Figure 9 is a diagram showing a ¹³C-NMR spectrum of Compound 17 (100 MHz, Pyr-d5).
Figure 10 is a diagram showing extracted ion chromatograms of Novel steviol glycoside 1E (stevia leaf extract) and a chemically synthesized product (β-form of Reference Compound 15).
Figure 11 is a diagram showing MS/MS and MS³-fragmented mass spectra of Novel steviol glycoside 1E (stevia leaf extract) and the chemically synthesized product (β-form of Reference Compound 15).
Figure 12 is a diagram showing extracted ion chromatograms of Novel steviol glycoside 2E (stevia leaf extract) and a chemically synthesized product (β-form of Compound 17).
Figure 13 is a diagram showing MS/MS and MS³-fragmented mass spectra of Novel steviol glycoside 2E (stevia leaf extract) and the chemically synthesized product (β-form of Compound 17).
Figure 14 is a diagram showing results of sensory evaluations for comparison of Novel steviol glycoside A with sugar, rebaudioside A, rebaudioside D and rebaudioside M.
Figure 15 is a diagram showing results of sensory evaluations for comparison of Novel steviol glycoside B with sugar, rebaudioside A, rebaudioside D and rebaudioside M.
Figure 16 is a diagram showing results of an expression analysis in relation to identification of the genetic features in the example.

### Description of Embodiments

Hereinafter, the present invention will be described in detail. The following embodiment is provided for illustrating the present invention with no intention of limiting the present invention solely to this embodiment. The present invention may be carried out in various modes without departing from the scope thereof. The present specification incorporates the contents of the specification and the drawings of Japanese Patent Application No. 2019-141627, filed on July 31, 2019, from which the present application claims priority.

The terms "rebaudioside", "Reb" and "Reb." as used herein have the same meaning and all of them refer to "rebaudioside". Similarly, the term "dulcoside" as used herein refers to "dulcoside".

### 1. Novel steviol glycoside

For the first time, the present inventors identified the structure of a novel steviol glycoside that contains xylose. The novel steviol glycoside of the present invention (herein, also referred to as the "glycoside of the present invention") is a compound represented by Formula (3), or a salt or a hydrate thereof:

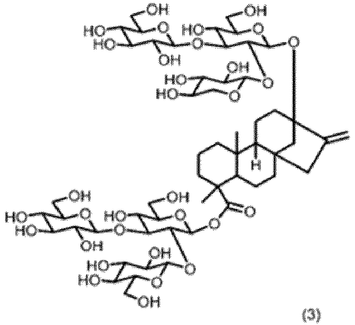

As represented above, the glycoside of the present invention comprises a steviol glycoside having a sugar chain containing two glucose moieties and one xylose moiety at C-13 of steviol and a sugar chain containing three glucose moieties at C-19 of steviol (herein, also referred to as "Glycoside B of the present invention").

Furthermore, as used herein, Glc represents glucose and Xyl represents xylose. As used herein, "Glc" may be α- or β-glucose while Xyl may be α- or β-xylose. Alternatively, as used herein, Glc may be α- and β-glucose while Xyl may be α- and β-xylose. Moreover, "Glc1-" indicates that the carbon atom at C-1 of glucose is attached to steviol via a glycosidic bond, and "Glc(1-3)-Glc1-" indicates that the carbon atom at C-3 of glucose represented by "Glc1-" is attached to a carbon atom at C-1 of another glucose via a glycosidic bond. Furthermore, "Xyl(1-2)-Glc1-" indicates that the carbon atom at C-2 of glucose represented by "Glc1-" is attached to the carbon atom at C-1 of xylose via a glycosidic bond. Furthermore, "Xyl(1-2)[Glc(1-3)]Glc1-" indicates that the carbon atom at C-2 of glucose represented by "Glc1-" is attached to the carbon atom at C-1 of xylose via a glycosidic bond, and the carbon atom at C-3 of glucose represented by "Glc1-" is attached to the carbon atom at C-1 of glucose via a glycosidic bond.

Examples of Glycoside B include glycosides having the structures represented by Formulae (3) and (5) .

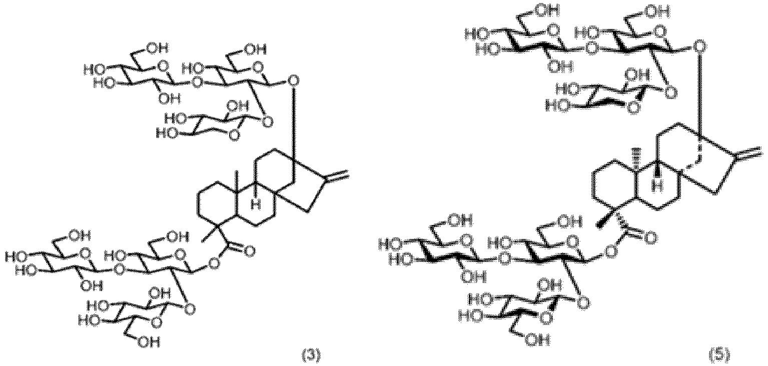

In Glycoside B represented by Formula (3), glucose is attached to the hydroxy group at C-13 of steviol via a β-glycosidic bond, another glucose is attached to said glucose via a β1-3 bond and xylose is attached to said glucose via a β1-2 bond. Formulae (5) represents a structure having further specified conformations of Glycoside B represented by Formulae (3).

The glycoside of the present invention also comprises isomers such as the α- and β-forms as described above. Therefore, the glycoside of the present invention may comprise only the α-form, only the β-form or a mixture of the α- and β-forms. The proportion of the β-form in the glycoside of the present invention is preferably 80% or more, more preferably 90% or more, still more preferably 95% or more and particularly preferably 99% or more. The α- and β-forms can be isolated/purified by a known method such as high-performance liquid chromatography (HPLC), or ultra (high) performance liquid chromatography (UPLC).

The glycoside of the present invention may not only be the compound represented by Formula (3) but may also be a derivative, a salt or a hydrate thereof. The term "derivative" as used herein refers to a compound resulting from a structural change of a minor moiety of the compound, for example, a compound in which some of the hydroxy groups are substituted with other substituents. Therefore, derivatives of the compound represented by Formula (3) include compounds in which some of the hydroxy groups contained in the compound have been substituted with a substituent selected from hydrogen, a halogen, an alkyl group, an alkenyl group, an alkynyl group, an aryl group, an amino group, or a cyano group. As used herein, a "salt of the compound represented by Formula (3)" refers to a physiologically acceptable salt, for example, a sodium salt, of the compound represented by Formula (3). Furthermore, a "hydrate of the compound represented by Formula (3)" as used herein refers to a compound resulting from attachment of a water molecule to the compound represented by Formula (3).

While the glycoside of the present invention is not particularly limited, it may be a plant-derived product, a chemically synthesized product or a biosynthetic product. For example, it may be isolated and purified from a plant rich in the glycoside of the present invention, or it may be obtained by a chemical synthesis or a biosynthesis. Details of a method for producing the glycoside of the present invention will be described later herein.

The glycoside of the present invention is sweeter than sugar (sucrose), and can affect the sweetness of a food or beverage in a small amount. Thus, the glycoside of the present invention can be used as a novel sweetener.

A glycoside of the present invention is Glycoside B. Glycoside B is sweeter than sugar, and has weaker bitterness than other steviol glycosides. Accordingly, the steviol glycoside of the present invention can favorably be used as a sweetener in various applications as will be described later.

### 2. Sweetener composition comprising novel steviol glycoside

In one aspect of the present invention, a sweetener composition comprising the compound represented by Formula (3), or a derivative, a salt or a hydrate thereof (hereinafter, also referred to as the "sweetener composition of the present invention") is provided. The sweetener composition of the present invention is not particularly limited as long as it contains the compound represented by Formula (3), or a derivative, a salt or a hydrate thereof, and it may be a composition comprising an extract comprising the compound represented by Formula (3), or a derivative, a salt or a hydrate thereof.

The amount of the glycoside of the present invention contained in the sweetener composition of the present invention is not particularly limited, and may be, for example, 1-99 wt%, 5-95 wt%, 10-90 wt%, 15-85 wt%, 20-80 wt%, 25-75 wt%, 30-70 wt%, 35-65 wt%, 40-60 wt%, 45-55 wt%, 1-5 wt%, 1-10 wt%, 1-15 wt%, 1-20 wt%, 1-25 wt%, 1-30 wt%, 1-35 wt%, 1-40 wt%, 1-45 wt% or 1-50 wt% relative to the total amount of the sweetener composition.

The sweetener composition of the present invention may further contain other steviol glycosides. For example, the sweetener composition of the present invention may contain, in addition to the glycoside of the present invention, one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside.

In a case where other steviol glycoside is contained, the composition ratio of the glycoside of the present invention and other steviol glycoside may be 1:99 to 99:1, 5:95 to 95:5, 10:90 to 90:10, 15:85 to 85:15, 20:80 to 80:20, 25:75 to 75:25, 30:70 to 70:30, 35:65 to 65:35, 40:60 to 60:40, 45:65 to 65:45 or 50:50 in a mass ratio. Moreover, either a single or multiple types of glycosides of the present invention may be used.

The sweetener composition of the present invention may further contain a sweetener other than the steviol glycosides. Examples of such a sweetener include natural sweeteners such as fructose, sugar, fructose-glucose syrup, glucose, maltose, high-fructose syrup, sugar alcohol, oligosaccharide, honey, pressed sugarcane juice (brown sugar syrup), starch syrup, Lo Han Kuo *(Siraitia grosvenorii)* powder, a Lo Han Kuo *(Siraitia grosvenorii)* extract, licorice powder, a licorice extract, *Thaumatococcus daniellii* seed powder, a *Thaumatococcus daniellii* seed extract, and artificial sweeteners such as acesulfame potassium, sucralose, neotame, aspartame and saccharin. Among them, a natural sweetener is preferably used from the aspect of imparting clean taste, easy drinkability, natural flavor and moderately rich taste, where fructose, glucose, maltose, sucrose and sugar are particularly preferably used. Either a single or multiple types of these sweetness components may be used.

A method for producing the sweetener composition of the present invention is not particularly limited as long as a sweetener composition having the above-described composition can be obtained. In one aspect of the present invention, a method for producing a sweetener composition of the present invention, the method comprising the steps of: obtaining a glycoside of the present invention; and mixing the glycoside with other steviol glycoside or a sweetener other than a steviol glycoside, is provided. The step of obtaining a glycoside of the present invention may be carried out by isolation/purification from a plant, a chemical synthesis or a biosynthesis, where the glycoside of the present invention resulting from this step may be obtained as a mixture with other steviol glycoside (for example, Reb.A or Reb.D).

### 3. Food or beverage, flavoring agent and pharmaceutical product comprising novel steviol glycoside

In one aspect of the present invention, a food or beverage, a flavoring agent and a pharmaceutical product comprising the compound represented by Formula (3) or a derivative, a salt or a hydrate thereof or the sweetener composition of the present invention (herein, also referred to as a "food or beverage of the present invention", a "flavoring agent of the present invention" and a "pharmaceutical product of the present invention", respectively) are provided. The food or beverage, the flavoring agent and the pharmaceutical product of the present invention are not particularly limited as long as they contain the compound represented by Formula (3), or a derivative, a salt or a hydrate thereof, and they may be a food or beverage, a flavoring agent and a pharmaceutical product comprising an extract or a sweetener composition comprising the compound represented by Formula (3), or a derivative, a salt or a hydrate thereof. Herein, a food or beverage refers to beverages and foods. Therefore, in some embodiments, the present invention provides a novel beverage or food, and a method for producing said beverage or food.

While the amount of the glycoside of the present invention contained in the food or beverage, the flavoring agent and the pharmaceutical product of the present invention differs depending on the specific food or beverage, it is preferably around 1-600 mass ppm, for example, 20-550 mass ppm, 25-550 mass ppm, 30-550 mass ppm, 35-550 mass ppm, 40-550 mass ppm, 45-550 mass ppm, 50-550 mass ppm, 55-550 mass ppm, 20-540 mass ppm, 25-540 mass ppm, 30-540 mass ppm, 35-540 mass ppm, 40-540 mass ppm, 45-540 mass ppm, 50-540 mass ppm, 55-540 mass ppm, 20-530 mass ppm, 25-530 mass ppm, 30-530 mass ppm, 35-530 mass ppm, 40-530 mass ppm, 45-530 mass ppm, 50-530 mass ppm, 55-530 mass ppm, 20-520 mass ppm, 25-520 mass ppm, 30-520 mass ppm, 35-520 mass ppm, 40-520 mass ppm, 45-520 mass ppm, 50-520 mass ppm, 55-520 mass ppm, 20-510 mass ppm, 25-510 mass ppm, 30-510 mass ppm, 35-510 mass ppm, 40-510 mass ppm, 45-510 mass ppm, 50-510 mass ppm, 55-510 mass ppm, 20-505 mass ppm, 25-505 mass ppm, 30-505 mass ppm, 35-505 mass ppm, 40-505 mass ppm, 45-505 mass ppm, 50-505 mass ppm, 55-505 mass ppm, 20-500 mass ppm, 25-500 mass ppm, 30-500 mass ppm, 35-500 mass ppm, 40-500 mass ppm, 45-500 mass ppm, 50-500 mass ppm, 55-500 mass ppm, 20-495 mass ppm, 25-495 mass ppm, 30-495 mass ppm, 35-495 mass ppm, 40-495 mass ppm, 45-495 mass ppm, 50-495 mass ppm, 55-495 mass ppm, 20-490 mass ppm, 25-490 mass ppm, 30-490 mass ppm, 35-490 mass ppm, 40-490 mass ppm, 45-490 mass ppm, 50-490 mass ppm, 55-490 mass ppm, 100-400 mass ppm, 150-400 mass ppm, 200-400 mass ppm, 250-400 mass ppm, 300-400 mass ppm, 100-150 mass ppm, 100-200 mass ppm, 100-250 mass ppm or 100-300 mass ppm, in a case of a beverage. The content within this range is advantageous for imparting moderate sweetness. The content within this range is advantageous for suppressing the lingering aftertaste. Unless otherwise specified, "ppm" as used herein refers to "mass ppm".

The food or beverage, the flavoring agent and the pharmaceutical product of the present invention may further contain other steviol glycosides. For example, the sweetener composition of the present invention may contain, in addition to the glycoside of the present invention, one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside.

In a case where other steviol glycoside is contained, the composition ratio of the glycoside of the present invention and other steviol glycoside may be 1:99 to 99:1, 5:99 to 95:5, 10:90 to 90:10, 15:85 to 85:15, 20:80 to 80:20, 25:75 to 75:25, 30:70 to 70:30, 35:65 to 65:35, 40:60 to 60:40, 45:65 to 65:45 or 50:50 in a mass ratio.

The food or beverage, the flavoring agent and the pharmaceutical product of the present invention may further contain a sweetener other than a steviol glycoside. Examples of such a sweetener include natural sweeteners such as fructose, sugar, fructose-glucose syrup, glucose, maltose, sucrose, high-fructose syrup, sugar alcohol, oligosaccharide, honey, pressed sugarcane juice (brown sugar syrup), starch syrup, Lo Han Kuo *(Siraitia grosvenorii)* powder, a Lo Han Kuo *(Siraitia grosvenorii)* extract, licorice powder, a licorice extract, *Thaumatococcus daniellii* seed powder and a *Thaumatococcus daniellii* seed extract, and artificial sweeteners such as acesulfame potassium, sucralose, neotame, aspartame and saccharin. Among them, a natural sweetener is preferably used from the aspect of imparting clean taste, easy drinkability, natural flavor and moderately rich taste, where fructose, glucose, maltose, sucrose and sugar are particularly preferably used. Either a single or multiple types of these sweetness components may be used.

Examples of the food of the present invention include, but not particularly limited to, a confection, a bread, cereal flour, noodles, rice, a processed agricultural/forestry food, a processed livestock product, a processed fishery product, a milk/dairy product, an oil-and-fat/processed oil-and-fat product, seasoning or other food materials.

Examples of the beverage of the present invention include, but not particularly limited to, a carbonated beverage, a non-carbonated beverage, an alcoholic beverage, a non-alcoholic beverage, a beer-taste beverage such as beer or non-alcohol beer, a coffee beverage, a tea beverage, a cocoa beverage, a nutritious beverage and a functional beverage.

The beverage of the present invention may be heat sterilized and packaged to be prepared as a packaged beverage. Examples of such a package include, but not particularly limited to, a PET bottle, an aluminum can, a steel can, a paper package, a chilled cup and a bottle. If heat sterilization is to be performed, the type of heat sterilization is not particularly limited, and heat sterilization may be performed, for example, by employing a common technique such as UHT sterilization, or retort sterilization. While the temperature during the heat sterilization process is not particularly limited, it is, for example, 65-130°C, preferably 85-120°C for 10-40 minutes. Sterilization, however, can be carried out at an appropriate temperature for a several seconds, for example, 5-30 seconds, without any problem as long as a sterilizing value comparative to that under the above-described conditions can be earned.

The method for producing the food or beverage, the flavoring agent and the pharmaceutical product of the present invention is not particularly limited as long as a food or beverage, a flavoring agent and a pharmaceutical product having the above-described components can be obtained. In one aspect of the present invention, a method for producing a food or beverage, a flavoring agent and a pharmaceutical product of the present invention, the method comprising the steps of: obtaining the extract, the glycoside or the sweetener composition of the present invention; and adding the extract, the glycoside or the sweetener composition to a food or beverage, a flavoring agent, a pharmaceutical product or a raw material thereof, is provided. The step of obtaining the glycoside or the sweetener composition of the present invention and the step of obtaining the extract of the present invention are described in "2. Sweetener composition comprising novel steviol glycoside" and "4. Stevia plant comprising novel steviol glycoside and extract thereof", respectively. The step of adding the extract, the glycoside or the sweetener composition of the present invention to a food or beverage, a flavoring agent, a pharmaceutical product or a raw material thereof can be carried out during any step of producing the food or beverage, the flavoring agent and the pharmaceutical product. For example, it may be carried out upon mixing the raw materials of the food or beverage, the flavoring agent or the pharmaceutical product, or upon the final adjustments of the taste quality of the food or beverage, the flavoring agent or the pharmaceutical product.

### 4. Stevia plant comprising novel steviol glycoside and extract thereof

Described herein is a stevia plant comprising the compound represented by Formula (3), or a derivative, a salt or a hydrate thereof and an extract thereof (herein, also referred to as "the plant described herein and the extract thereof"). Further described is a food or beverage, a flavoring agent and a pharmaceutical product, preferably a beverage, comprising the described plant or the extract thereof. While the amount of the glycoside of the present invention contained in the plant described herein is not particularly limited, it may be 0.001-1.000 wt%, 0.005-0.950 wt%, 0.008-0.900 wt%, 0.010-0.850 wt% or 0.015-0.800 wt% in a dried leaf.

The plant described hereinhas at least one of the genetic features (1) to (8) below:
(1) being homozygous or heterozygous for the allele wherein the base at the position corresponding to position 298 of SEQ ID NO:1, position 11 of SEQ ID NO:3, position 21of SEQ ID NO:5 or position 26 of SEQ ID NO:7 is T;
(2) being homozygous or heterozygous for the allele wherein the base at the position corresponding to position 328 of SEQ ID NO:1, position 11 of SEQ ID NO:9, position 21 of SEQ ID NO:11 or position 26 of SEQ ID NO:13 is C;
(3) being homozygous or heterozygous for the allele wherein the base at the position corresponding to position 360 of SEQ ID NO:1, position 11 of SEQ ID NO:15, position 21 of SEQ ID NO:17 or position 26 of SEQ ID NO:19 is T;
(4) being homozygous or heterozygous for the allele wherein the base at the position corresponding to position 386 of SEQ ID NO:1, position 11 of SEQ ID NO:21, position 21 of SEQ ID NO:23 or position 26 of SEQ ID NO:25 is T;
(5) being homozygous or heterozygous for the allele wherein the base at the position corresponding to position 393 of SEQ ID NO:1, position 11 of SEQ ID NO:27, position 18 of SEQ ID NO:29 or position 23 of SEQ ID NO:31 is T;
(6) being homozygous or heterozygous for the allele wherein the base at the position corresponding to position 411 of SEQ ID NO:1, position 11 of SEQ ID NO:33, position 18 of SEQ ID NO:35 or position 23 of SEQ ID NO:37 is T;
(7) being homozygous or heterozygous for the allele wherein the base at the position corresponding to position 427 of SEQ ID NO:1, position 11 of SEQ ID NO:39, position 18 of SEQ ID NO:41 or position 23 of SEQ ID NO:43 is C; and
(8) being homozygous or heterozygous for the allele wherein the base at the position corresponding to position 453 of SEQ ID NO:1, position 11 of SEQ ID NO:45, position 18 of SEQ ID NO:47 or position 23 of SEQ ID NO:49 is T.

The plant described herein has at least two, at least three, at least four, at least five, at least six, at least seven or all of the eight genetic features (1) to (8).

The phrase "position (or a part) corresponding to" refers to the position or the part (for example, position 298, position 328, position 360, position 386, position 393, position 411, position 427 and position 453, etc.) of the sequence existing in the genome if said sequence existing in the genome is identical to the reference sequence (for example, SEQ ID NO:1, etc.). If none of the sequences existing in the genome is identical to the reference sequence, the phrase refers to a position or a part of a sequence existing in the genome which correlates with the position or the part of the reference sequence. Whether or not a sequence identical to or correlating with the reference sequence exists in the genome can be determined, for example, as follows: the genomic DNA of the intended stevia plant is amplified with primers that can amplify the reference sequence through PCR; the amplified product is sequenced; and an alignment analysis is performed between the resulting sequence and the reference sequence. Examples of sequences corresponding to the reference sequence include, but not limited to, nucleotide sequences having sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more or 99.8% or more to the reference sequence. A position or a part of a sequence existing in the genome which correlates with the position or the part of the reference sequence can be determined by considering the nucleotide sequences preceding and following the position or the part of the reference sequence. For example, the reference sequence can be aligned with a sequence in the genome corresponding to the reference sequence so as to determine the position or the part of the sequence existing in the genome which correlates with the position or the part of the reference sequence.

For example, in the case of "the position corresponding to position 298 of SEQ ID NO:1" as in the genetic feature (1) of the plant described herein, if the genome of the stevia plant has a part consisting of a nucleotide sequence identical to SEQ ID NO:1, "the position corresponding to position 298 of SEQ ID NO:1" refers to position 298 from the 5' end of said part of the genome, which consists of a nucleotide sequence identical to SEQ ID NO:1. Meanwhile, if the genome of the stevia plant has a part consisting of a nucleotide sequence that is not identical but that corresponds to SEQ ID NO:1, "the position corresponding to position 298 of SEQ ID NO:1" does not necessarily refers to the 298th position from the 5' end of the part corresponding to SEQ ID NO:1 since the genome does not have a part consisting of a nucleotide sequence that is identical to SEQ ID NO:1, but "the position corresponding to position 298 of SEQ ID NO:1" in said genome of the stevia plant can be specified by considering the nucleotide sequences preceding or following position 298 of SEQ ID NO:1. For example, "the position corresponding to position 298 of SEQ ID NO:1" in the genome of the stevia plant can be specified by an alignment analysis between a nucleotide sequence in the genome of the stevia plant, which corresponds to SEQ ID NO:1 and the nucleotide sequence of SEQ ID NO:1.

While the aforementioned genetic features can be detected by PCR method, TaqMan PCR method, sequencing method, microarray method, Invader assay, TILLING assay, RAD (random amplified polymorphic DNA) method, restriction fragment length polymorphism (RFLP) method, PCR-SSCP method, AFLP (amplified fragment length polymorphism) method, SSLP (simple sequence length polymorphism) method, CAPS (cleaved amplified polymorphic sequence) method, dCAPS (derived cleaved amplified polymorphic sequence) method, allele-specific oligonucleotide (ASO) method, ARMS method, denaturing gradient gel electrophoresis (DGGE) method, CCM (chemical cleavage of mismatch) method, DOL method, MALDI-TOF/MS method, TDI method, padlock probe assay, molecular beacon assay, DASH (dynamic allele specific hybridization) method, UCAN method, ECA method, PINPOINT method, PROBE (primer oligo base extension) method, VSET (very short extension) method, Survivor assay, Sniper assay, Luminex assay, GOOD method, LCx method, SNaPshot method, Mass ARRAY assay, pyrosequencing method, SNP-IT method, or melting curve analysis, the detection method is not limited thereto.

In one description, the "part consisting of a nucleotide sequence corresponding to SEQ ID NO: 1" may include, for example, a part of the genome of the stevia plant which can be amplified by PCR using a forward primer containing the nucleotide sequence represented by SEQ ID NO:51 and a reverse primer containing the nucleotide sequence represented by SEQ ID NO:52.

In one description, an "allele wherein the base at the position corresponding to position 298 of SEQ ID NO:1 is T" includes the nucleotide sequence represented by SEQ ID NO:4, 6 or 8.

In one description,, an "allele wherein the base at the position corresponding to position 328 of SEQ ID NO:1 is C" includes the nucleotide sequence represented by SEQ ID NO: 10, 12 or 14.

In one description,, an "allele wherein the base at the position corresponding to position 360 of SEQ ID NO:1 is T" includes the nucleotide sequence represented by SEQ ID NO:16, 18 or 20.

In one description,, an "allele wherein the base at the position corresponding to position 386 of SEQ ID NO:1 is T" includes the nucleotide sequence represented by SEQ ID NO:22, 24 or 26.

In one description,, an "allele wherein the base at the position corresponding to position 393 of SEQ ID NO:1 is T" includes the nucleotide sequence represented by SEQ ID NO:28, 30 or 32.

In one description,, an "allele wherein the base at the position corresponding to position 411 of SEQ ID NO:1 is T" includes the nucleotide sequence represented by SEQ ID NO:34, 36 or 38.

In one description,, an "allele wherein the base at the position corresponding to position 427 of SEQ ID NO:1 is C" includes the nucleotide sequence represented by SEQ ID NO:40, 42 or 44.

In one description,, an "allele wherein the base at the position corresponding to position 453 of SEQ ID NO:1 is T" includes the nucleotide sequence represented by SEQ ID NO:46, 48 or 50.

While a method for producing the plant described herein is not particularly limited, it may be created by crossbreeding, or by introducing a mutation described herein into the genome of the stevia plant. The introduction of the variation may be performed by a genetic modification approach or may be performed by a non-genetic modification approach. Descriptive examples of the "non-genetic modification approach" include a method of inducing a variation in the gene of a host cell (or a host plant) without transfection with a foreign gene. Descriptive examples of such a method include a method of allowing a mutagen to act on a plant cell. Descriptive examples of such a mutagen include ethylmethanesulfonic acid (EMS) and sodium azide. For example, ethylmethanesulfonic acid (EMS) can be used at a concentration such as 0.1%, 0.2%, 0.3%, 0.4%, 0.5%, 0.6%, 0.7%, 0.8%, 0.9% or 1.0% to treat a plant cell. The treatment time is 1 to 48 hours, 2 to 36 hours, 3 to 30 hours, 4 to 28 hours, 5 to 26 hours, 6 to 24 hours. The procedures themselves of the treatment are known in the art and can be performed by dipping a water-absorbed seed obtained through a water absorption process in a treatment solution containing the mutagen at the concentration described above for the treatment time described above.

An alternative descriptive example of the non-genetic modification approach can be a method of irradiating a plant cell with radiation or light beam such as X ray, γ ray, or ultraviolet ray. In this case, a cell irradiated using an appropriate dose (ultraviolet lamp intensity, distance, and time) of ultraviolet ray is cultured in a selective medium, and then, a cell, a callus, or a plant having the trait of interest can be selected. In this operation, the irradiation intensity is 0.01 to 100 Gr, 0.03 to 75 Gr, 0.05 to 50 Gr, 0.07 to 25 Gr, 0.09 to 20 Gr, 0.1 to 15 Gr, 0.1 to 10 Gr, 0.5 to 10 Gr, 1 to 10 Gr. The irradiation distance is 1 cm to 200 m, 5 cm to 100 m, 7 cm to 75 m, 9 cm to 50 m, 10 cm to 30 m, 10 cm to 20 m, 10 cm to 10 m. The irradiation time is 1 minute to 2 years, 2 minutes to 1 year, 3 minutes to 0.5 years, 4 minutes to 1 month, 5 minutes to 2 weeks, or 10 minutes to 1 week. The irradiation intensity, distance and time differ depending on the type of radiation or the state of the subject to be irradiated (cell, callus, or plant) and can be appropriately adjusted by those skilled in the art.

Furthermore, the phrase "0.050 wt% or more relative to the amount of the total steviol glycosides contained in the leaf" means that the glycoside of the present invention exists at a percentage of 0.050 wt% or more with respect to the amount of the total steviol glycosides existing in the liquid extract derived from the dried leaf of the stevia plant described herein. Here, the total steviol glycosides does not contain any unknown steviol glycoside or any steviol glycoside existing in an amount less than the detection limit. Preferably, the total steviol glycosides consist of rebaudioside A, rebaudioside B, rebaudioside C, rebaudioside D, rebaudioside E, rebaudioside F, rebaudioside G, rebaudioside I, rebaudioside M, rebaudioside N, stevioside, dulcoside A, steviol bioside, rubusoside and novel steviol glycosides (reference steviol glycoside A and/or Novel steviol glycoside B). The content of reference steviol glycoside A or novel steviol glycoside B in the leaf of the stevia plant described herein may be 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total steviol glycosides.

Alternatively, the content of reference steviol glycoside A or novel steviol glycoside B in the leaf of the stevia plant described herein may be 0.050 wt% or more, 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total content of rebaudioside A, rebaudioside B, rebaudioside C, rebaudioside D, rebaudioside F, rebaudioside N, rebaudioside M and stevioside contained in the leaf.

Here, the dried leaf of the plant described herein refer to those obtained by drying a fresh leaf of the plant described herein to reduce their water content to be 10 wt% or less, 7 wt% or less, 5 wt% or less, 4 wt% or less, 3 wt% or less, 2 wt% or less, or 1 wt% or less. Preferably, the water content of the dried leaf of the plant described herein is 3-4 wt%.

The plant desribed herein not only comprises the whole plant but may also comprise plant organs (for example, leaf, petal, stem, root, seed, etc.), plant tissues (for example, epidermis, phloem, parenchyma, xylem, vascular bundles, palisade tissue, spongy tissue, etc.), various forms of plant cells (for example, suspension cultured cells), a protoplast, a leaf piece, and callus.

In addition, the plant described herein may also comprise a tissue culture or a plant cell culture. This is because such a tissue culture or plant cell culture can be cultured to regenerate a plant. Descriptive examples of the tissue culture or the plant cell culture of the plant described herein include, but not limited to, an embryo, meristematic cells, pollen, a leaf, a root, a root apex, a petal, a protoplast, a leaf piece and callus.

An extract of the plant described herein can be obtained by allowing a fresh or dried leaf of the plant of the present invention to react with an appropriate solvent (an aqueous solvent such as water or an organic solvent such as alcohol, ether or acetone). For the extraction conditions, see the method described in WO2016/090460 or the method described in the example below.

Preferably, the extract of the plant described herein contains the glycoside of the present invention for 0.050 wt% or more relative to the total steviol glycosides. The content of the glycoside of the present invention may be 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total steviol glycosides.

Alternatively, the content of reference steviol glycoside A or novel steviol glycoside in the extract of the plant described herein may be 0.050 wt% or more, 0.055 wt% or more, 0.060 wt% or more, 0.065 wt% or more, 0.070 wt% or more, 0.075 wt% or more, 0.080 wt% or more, 0.085 wt% or more, 0.090 wt% or more, 0.095 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more or 10.00 wt% or more, and 10.00 wt% or less, 8.00 wt% or less, 6.00 wt% or less, 4.00 wt% or less, 2.00 wt% or less, 1.00 wt% or less, 0.80 wt% or less, 0.60 wt% or less or 0.30 wt% or less, relative to the total content of rebaudioside A, rebaudioside B, rebaudioside C, rebaudioside D, rebaudioside F, rebaudioside N, rebaudioside M and stevioside.

Described here is a food or beverage comprising the extract of the plant described herein. As described here the food or beverage is a beverage. Examples of the kinds of the food or beverage include those recited in "3. Food or beverage comprising novel steviol glycoside".

### 5. Flavor controlling agent comprising novel steviol glycoside

In one aspect of the present invention, a flavor controlling agent comprising the above-described compound represented by Formula (3), or a derivative, a salt or a hydrate thereof is provided. In one aspect of the present invention, a composition composed as described in "2. Sweetener composition comprising novel steviol glycoside" may also be used as a flavor controlling agent.

Herein, a "flavor controlling agent" refers to a substance that can be added to a food or beverage to control the flavor of the food or beverage. Preferably, the flavor controlling agent of the present invention can be added to a food or beverage so as to control the flavor of the food or beverage itself without the consumers recognizing the taste of the flavor controlling agent itself. For example, since the steviol glycoside of the present invention has weaker bitterness as compared to conventional steviol glycosides, it can be used as a flavor controlling agent for controlling the bitterness of the food or beverage.

In addition to the above-described compound represented by Formula (3) or a derivative, a salt or a hydrate thereof, the flavor controlling agent of the present invention preferably comprises one or more types of other sweeteners. Examples of such sweetener include: one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside; natural sweeteners such as fructose, sugar, fructose-glucose syrup, glucose, maltose, sucrose, high-fructose syrup, sugar alcohol, oligosaccharide, honey, pressed sugarcane juice (brown sugar syrup), starch syrup, Lo Han Kuo *(Siraitia grosvenorii)* powder, a Lo Han Kuo (*Siraitia grosvenorii*) extract, licorice powder, a licorice extract, *Thaumatococcus daniellii* seed powder and a *Thaumatococcus daniellii* seed extract; and artificial sweeteners such as acesulfame potassium, sucralose, neotame, aspartame and saccharin.

### 6. Method for producing novel steviol glycoside

As described above, the steviol glycoside of the present invention can be produced by (A) isolation/purification from a plant, (B) a chemical synthesis, or (C) a biosynthesis. Hereinafter, each of them will be described.

### (A) Isolation/purification from plant

A plant containing the novel steviol glycoside is obtained, for example, by obtaining a plant having the aforementioned genetic features by any known screening method. Thereafter, the novel steviol glycoside can be isolated/purified from the plant. A fresh or dried leaf of the plant of the present invention is allowed to react with an appropriate solvent (an aqueous solvent such as water or an organic solvent such as alcohol, ether or acetone) to extract the novel steviol glycoside in a liquid extract state. For extraction conditions and else, see the method described in WO2016/090460 or the method described in the example below.

Furthermore, the resulting liquid extract may be subjected to a known method such as a gradient of ethyl acetate or other organic solvent: water, high performance liquid chromatography (HPLC) or ultra (high) performance liquid chromatography (UPLC) to isolate/purify the novel steviol glycoside.

The content of the novel steviol glycoside in the plant can be determined by the method described in WO2016/090460 or the method described in the example below. Specifically, the content can be determined by sampling a fresh leaf from the plant of the present invention and subjecting the leaf to LC-MS/MS.

### (2) Chemical synthesis

The steviol glycoside of the present invention can be synthesized by a known chemical synthesis such as one described in WO2018/181515. More specifically, the steviol glycoside of the present invention can be produced by the method described in the example below.

### (3) Biosynthesis

The steviol glycoside of the present invention can also be generated by introducing a polynucleotide coding for a predetermined protein such as a glycosylation enzyme into a host cell derived from a bacterium, a plant, an insect, or a non-human mammal, and using steviol, a steviol glycoside, UDP-glucose and/or UDP-xylose as a substrate. Steviol, a steviol glycoside, UDP-glucose or UDP-xylose as the substrate may be either provided or biosynthesized in the cell.

Preferably, the polynucleotide coding for the predetermined protein is introduced into a host while being inserted into an appropriate expression vector. Such polynucleotides may individually be inserted into separate vectors.

An appropriate expression vector is generally made to contain:
(i) a promoter that allows transcription in the host cell;
(ii) a polynucleotide of the present invention linked to said promoter; and
(iii) an expression cassette that is involved in transcription termination and polyadenylation of RNA molecules and that contains, as a component thereof, a signal that functions in the host cell.

Examples of a method for preparing an expression vector include, but not particularly limited to, a method that uses a plasmid, a phage, or a cosmid, and DNA molecules having necessary components.

The specific type of the vector is not particularly limited, and any vector that allows expression in the host cell can suitably be selected. Specifically, a promoter sequence is suitably selected according to the type of the host cell to ensure the expression of the polynucleotide of the present invention, and a vector obtained by integrating this promoter sequence and the polynucleotide of the present invention into a plasmid is used as an expression vector.

The expression vector includes expression controlling regions (for example, a promoter, a terminator and/or an origin of replication) depending on the type of the host into which it is introduced. A promoter used in a bacterial expression vector may be a common promoter (for example, a trc promoter, a tac promoter, a lac promoter, etc.), a promoter used for a yeast may be, for example, a glyceraldehyde-3-phosphate dehydrogenase promoter, a PH05 promoter, or a GAL1/10 promoter, and a promoter for filamentous fungi may be, for example, amylase, or trpC. Moreover, examples of a promoter for expressing the gene of interest in a plant cell include a cauliflower mosaic virus 35S RNA promoter, a rd29A gene promoter, a rbcS promoter, and a mac-1 promoter in which the enhancer sequence of the cauliflower mosaic virus 35S RNA promoter is provided at the 5' end of a promoter sequence of *Agrobacterium-derived* mannopine synthase. A promoter for an animal cell host may be a viral promoter (for example, a SV40 early promoter, a SV40 late promoter, etc.). Examples of a promoter that is inducibly activated in response to external stimuli include a mouse mammary tumor virus (MMTV) promoter, a tetracycline responsive promoter, a metallothionein promoter and a heat shock protein promoter.

Preferably, the expression vector contains at least one selectable marker. As such a marker, an auxotrophic marker (LEU2, URA3, HIS3, TRP1, ura5, niaD), a drug resistance marker (hygromycin, zeocin), a geneticin resistance gene (G418r), a copper resistance gene (CUP1) (Marin et al., Proc. Natl. Acad. Sci. USA, vol. 81, p. 337, 1984), a cerulenin resistance gene (fas2m, PDR4) (Junji Inokoshi et al., Journal of Japanese Biochemical Society, vol. 64, p. 660, 1992; and Hussain et al., Gene, vol. 101, p. 149, 1991, respectively) can be used.

As a method for transforming a host cell, a generally employed known method can be employed. For example, transformation can be carried out by employing an electroporation method (Mackenxie, D. A. et al., Appl. Environ. Microbiol., vol. 66, p. 4655-4661, 2000), a particle delivery method (Japanese Unexamined Patent Application Publication No. 2005-287403), a spheroplast method (Proc. Natl. Acad. Sci. USA, vol. 75, p. 1929, 1978), a lithium acetate method (J. Bacteriology, vol. 153, p. 163, 1983), or a method described in Methods in yeast genetics, 2000 Edition: A Cold Spring Harbor Laboratory Course Manual, although the present invention is not limited thereto.

As to other general molecular biological processes, see "Sambrook and Russell, Molecular Cloning: A Laboratory Manual Vol. 3, Cold Spring Harbor Laboratory Press 2001", "Methods in Yeast Genetics, A laboratory manual (Cold Spring Harbor Laboratory Press, Cold Spring Harbor, NY)".

The non-human transformant obtained as described above can be cultured so as to allow the non-human transformant to produce the steviol glycoside. Such a non-human transformant is preferably a yeast. Moreover, the non-human transformant is preferably cultured in a medium containing steviol. The accumulated steviol glycoside can be extracted/purified to obtain the steviol glycoside of the present invention.

### Examples

Hereinafter, an example of the present invention will be described in detail, although the content of the present invention should not be limited thereto.

### [Isolation of novel steviol glycoside]

Two lines of novel stevia plants ((Cultivar A (EM3-4) and Cultivar B (EM2-27-15)) developed at Suntory Global Innovation Center (SIC) were prepared. These cultivars were obtained as follows. First, commercially available stevia seeds were sown and raised to subject the resulting seeds to genetic modification by a treatment with 0.2% (Cultivar B) or 0.3% (Cultivar A) ethyl methanesulfonate (EMS). The EMS-treated seeds were sown in the greenhouse at the Suntory World Research Center to obtain the original EMS-treated seedling (M0 generation). No difference in the germination rate was observed between the treatment concentrations. Cultivar A was derived from an individual of this M0 generation. Furthermore, the first treatment generation (M1 generation) seeds obtained by self-fertilization of all individuals of the M0 generation were collected and sown in the greenhouse at the Suntory World Research Center to obtain M1 generation seedlings. Cultivar B was derived from an individual of this M1 generation.

Extracts obtained from the leaf of Cultivars A and B were subjected to high performance liquid chromatography (HPLC) separation-mass spectrometry (MS) to perform the screening analysis of the steviol glycosides contained in the stevia plants based on the molecular weights of steviol glycosides formed of sugar chains including D-glucopyranosyl (Glc) and/or xylopyranosyl (Xyl). In this regard, Cultivars A and B were plants having the genetic features 1-8.

A process for preparing a test liquid was as follows: 10.0 mg each of lyophilized dried stevia leaves was weighed into a glass vial, to which 1.0 mL of water/methanol (1/1 vol/vol) was added as an extracting solvent, and then the resultant was subjected to ultrasonic irradiation in an ultrasonic cleaner (AS ONE, AS52GTU) at a set temperature of 25°C for 20 minutes, thereby obtaining liquid extracts of steviol glycosides from the stevia leaves. The resultant was further 10-fold diluted with water/methanol and filtrated through a filter with a pore size of 0.45 µm (Nacalai tesque, Cosmonice filter S (solvent)) so as to be subjected to HPLC-MS.

For the HPLC part of HPLC-MS, Nexera LC-30AD (Shimadzu Corporation) was used as a liquid delivery unit LC pump, and SM-C18 (4.6 x 250 mm) (from Imtakt) was used as a separation column. Liquid delivery of the LC mobile phase was carried out by using 0.2% acetic acid-containing Milli-Q water as mobile phase A and methanol as mobile phase B, where the binary gradient as follows: the concentration of the mobile phase B was constantly maintained at 10% for 0-5 minutes, allowed to shift from 10% to 70% in the next 15 minutes, then allowed to shift from 70% to 100% in the following 5 minutes, and maintained at 100% for 5 minutes at the end. The flow rate of the mobile phase was 0.4 mL/min, and 5 µL of the stevia leaf liquid extract that had been diluted and filtrated with a filter was injected.

For the MS part, triple quadrupole mass spectrometer LCMS-8030 (Shimadzu Corporation) equipped with an electrospray ionization (ESI) ion source was used. The mass spectrometry measurement was carried out in a selected ion monitoring (SIM) mode by selecting the negative ion measurement mode and the m/z values. The m/z values were selected by a calculation based on the molecular weights of steviol glycosides formed of sugar chains including D-glucopyranosyl (Glc) and/or xylopyranosyl (Xyl). Accordingly, m/z = 965.3 (Glc (4)), 1097.4 (Glc (4), Xyl (1)), 1127.4 (Glc (5)), 1259.5 (Glc (5), Xyl (1)), and 1289.5 (Glc (6)) were selected. Furthermore, a high purity reagent and rebaudiosides A, D and M that were available were also measured under the same conditions so as to confirm the negative ion m/z values and the retention time in HPLC. The peak areas (arbitrary unit) of the mainly detected steviol glycosides are shown in Table 1.

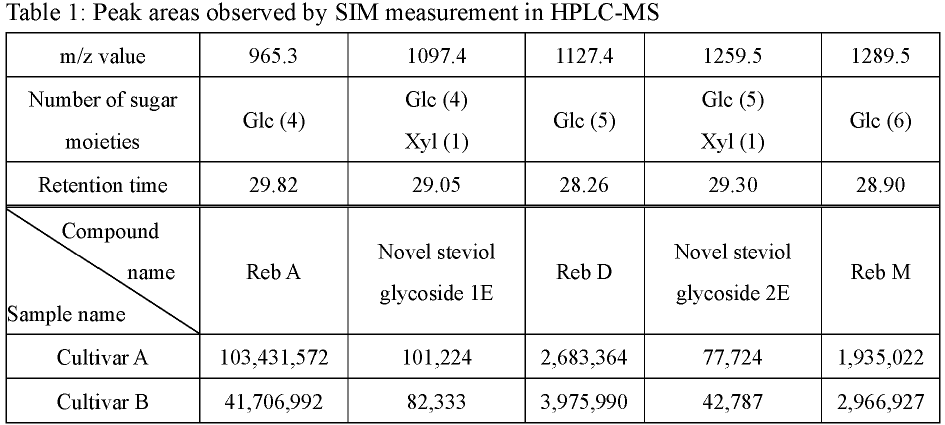

Figure 2 shows a selected ion chromatogram of Cultivar A at m/z of 1097.4. A peak of a molecular weight that had never been reported was observed in the selected ion chromatogram of a steviol glycoside (m/z 1097.4) in which the modified sugar chain contained four glucose moieties (Glc) and one xylose moiety (Xyl). Specifically, the peak at Rt 29.05 minutes shown in Figure 2 was an unknown substance. This substance was tentatively called "Novel steviol glycoside 1E". A similar peak was also detected for Cultivar B.

Figure 3 shows a selected ion chromatogram of Cultivar A at m/z of 1259.5. A peak of a molecular weight that had never been reported was observed in the selected ion chromatogram of a steviol glycoside (m/z 1259.5) in which the modified sugar chain contained five glucose moieties (Glc) and one xylose moiety (Xyl). Specifically, the peak at Rt 29.30 minutes shown in Figure 3 was an unknown substance. This substance was tentatively called "Novel steviol glycoside 2E". A similar peak was also detected for Cultivar B.

### [Structural analysis of novel steviol glycosides]

According to the present invention, structural analyses of Novel steviol glycosides 1E and 2E detected from the cultivars were performed in the following procedure.
(i) Structural deduction by a fragmentation analysis through high-performance liquid chromatography (HPLC)-high resolution mass spectrometry (MS), MS/MS, and three-stage ion fragmentation (MS³-fragmentation).
(ii) Chemical synthesis of the deduced steviol glycoside standard products via chemical reaction.
(iii) Structural determination by matching with the retention time and the fragmented pattern of the chemically synthesized standard product in HPLC-high resolution MS and MS³-fragmentation.

Hereinafter, each of Steps (i)-(iii) above will be described in detail.
(i) Structural deduction by a fragmentation analysis through high performance liquid chromatography (HPLC)-high resolution mass spectrometry (MS), MS/MS, and three-stage ion fragmentation (MS³-fragmentation)

Test liquids were prepared as follows: 10.0 mg each of lyophilized dried stevia leaves was weighed into a glass vial, to which 1.0 mL of water/methanol (1/1 vol/vol) was added as an extracting solvent, and then the resultant was subjected to ultrasonic irradiation in an ultrasonic cleaner (AS ONE, AS52GTU) at a set temperature of 25°C for 20 minutes, thereby obtaining liquid extracts of steviol glycosides from the stevia leaves. The resultant was further 10-fold diluted with water/methanol and filtrated through a filter with a pore size of 0.45 µm (Nacalai tesque, Cosmonice filter S (solvent system)) so as to be subjected to HPLC-MS.

In an equipment configuration for high performance liquid chromatography-electrospray ionization-high resolution mass spectrometry (HPLC-ESI-HRMS), the equipment for HPLC was configured by using Prominence LC-20AD (Shimadzu Corporation) as a liquid delivery unit LC pump and SM-C18 (4.6 x 250 mm) (from Imtakt) as a separation column. The LC mobile phase was delivered using 0.2% acetic acid-containing Milli-Q water as mobile phase A and methanol as mobile phase B, where the binary gradient was as follows: the concentration of the mobile phase B was constantly maintained at 10% for 0-5 minutes, then allowed to shift from 10% to 70% in the next 15 minutes, and further allowed to shift from 70% to 100% in the following 5 minutes. At the end, the concentration of the mobile phase B was maintained at 100% for 5 minutes. The flow rate of the mobile phase was 0.4 mL/min, and 20 µL of the stevia leaf liquid extract that had been diluted and subsequently filtrated with a filter was injected. For the mass spectrometry part, Orbitrap Elite MS (from Thermo Fisher Scientific) equipped with an ESI ion source was used. The mass spectrometry measurement was carried out in a negative ion measurement mode at m/z in a range of 150-2000 with resolution set to 60,000. The MS/MS measurement was carried out by selecting the targeted m/z of 1095.4 or 1257.5 and in a CID mode where fragmentation was induced by collision with an inert gas. The ion with the highest intensity in the MS/MS spectrum was targeted for MS³. Irradiation of energy required for fragmentation was performed at the standard collision energy unique to the apparatus, i.e., 35.

In order to study the fragmented patterns of Novel steviol glycosides 1E and 2E, standard samples, i.e., rebaudiosides A, D and M, having known structures were subjected to MS/MS and MS³-fragmentation pattern analyses. As a result, MS/MS of the novel steviol glycosides gave data showing that the highest ion intensity appeared at the peak where all sugar chains attached to C-19 via an ester bond were released. This result represents the total molecular weight of the sugar chains attached to the carbon of C-19 via an ester bond.

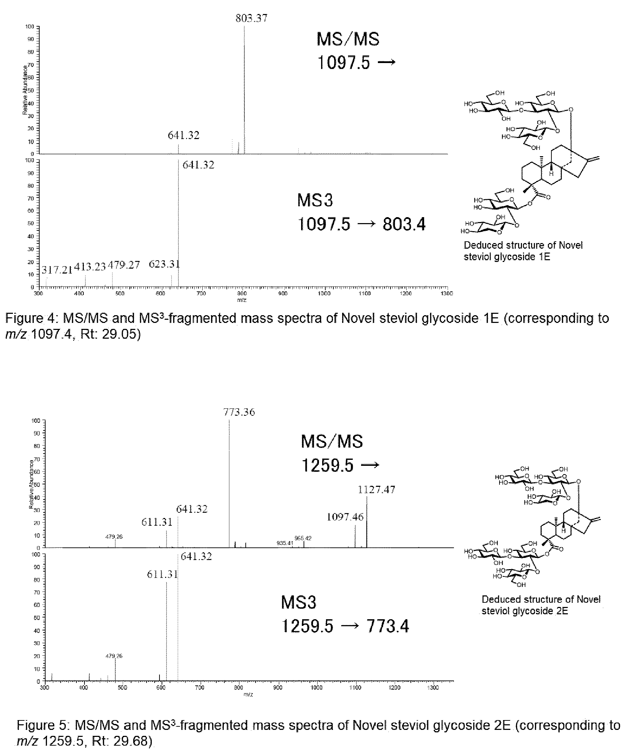

The MS/MS and MS³-fragmented mass spectra of Novel steviol glycoside 1E (corresponding to m/z 1097.4, Rt: 29.05) are shown in Figure 4. In the MS/MS spectrum of the novel steviol glycoside, the main peak was detected at m/z of 803.37 corresponding to the release of one Glc moiety and one Xyl moiety. From this result, the number of sugar chains attached to the carbon of C-19 via an ester bond was found to be one Glc moiety and one Xyl moiety. In order to acquire further structural information, a MS³ spectrum was acquired by fragmenting the main peak at m/z of 803.4 obtained by MS/MS. As a result, a spectrum having the same peak pattern as the MS³ spectrum of rebaudioside A (965.4→803.4→) was acquired. Accordingly, the sugar chains attached to C-13 were presumed to be the same as rebaudioside A. The deduced structure is shown in Figure 4.

The MS/MS and MS³-fragmented mass spectra of Novel steviol glycoside 2E (corresponding to m/z 1259.5, Rt: 29.30) are shown in Figure 5. In the MS/MS spectrum of the novel steviol glycoside, the main peak was detected at m/z of 773.36 corresponding to the release of three Glc moieties. From this result, the number of sugar chains attached to the carbon of C-19 via an ester bond was found to be three Glc moieties. In order to acquire further structural information, a MS³ spectrum was acquired by fragmenting the main peak at m/z of 773.4 obtained by MS/MS. As a result, a spectrum having the same peak pattern as the MS³ spectrum of rebaudioside F (935.4→773.4→) was acquired. Accordingly, the sugar chains attached to C-13 were presumed to be the same as rebaudioside F. The deduced structure is shown in Figure 5.

(ii) Chemical synthesis of the deduced steviol glycoside standard products (Novel steviol glycosides 1S and 2S) via chemical reaction

### [Synthesis of Novel steviol glycoside 1S]

### (1) Outline of synthetic pathways

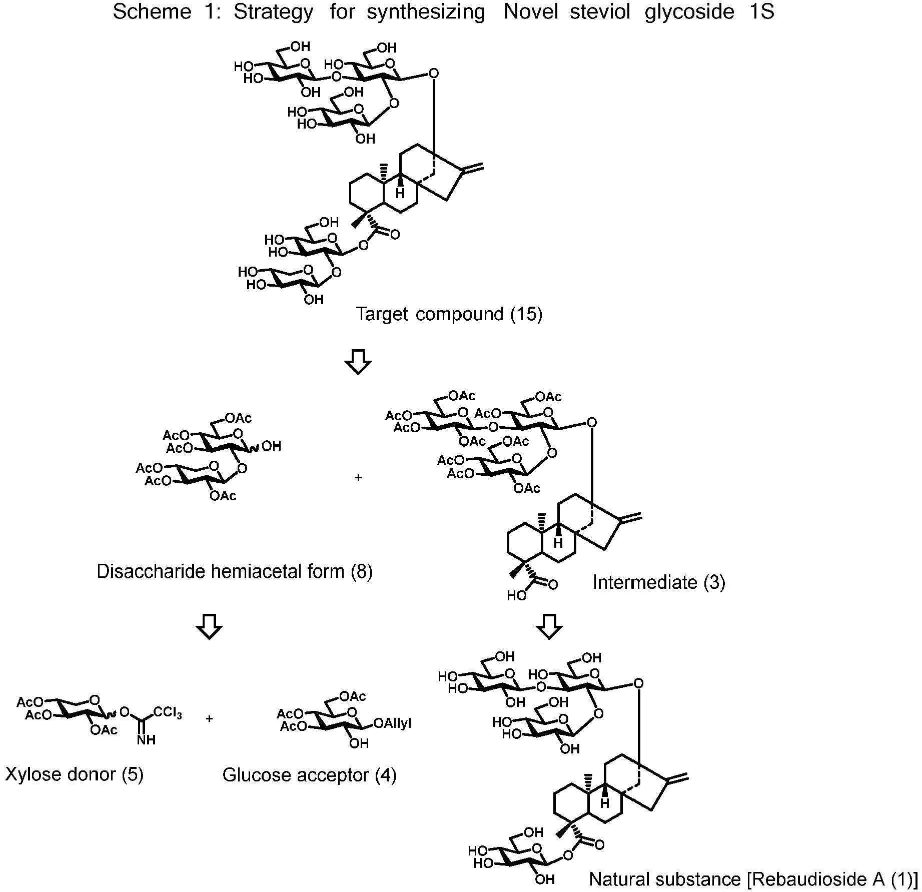

As can be appreciated from Scheme 1, for the synthesis of Novel steviol glycoside 1S (Reference compound 15), the intermediate (3) and the disaccharide hemiacetal form (8) were condensed via a Mitsunobu reaction to obtain the backbone of Novel steviol glycoside 1S (Reference compound 15). For the synthesis of the intermediate (3), the ester bond at C-19 of steviol of the known natural substance, i.e., rebaudioside A (1), was subjected to alkaline hydrolysis and then the hydroxy groups of the sugar chain were protected with acetyl (Ac) groups to obtain the intermediate (3). For the synthesis of the disaccharide hemiacetal form (8), a disaccharide backbone was produced through condensation reaction between an appropriately protected glucose acceptor (4) and a xylose donor (5), and the protecting group at the anomeric carbon of the reducing end was deprotected to give the disaccharide hemiacetal form (8). The resulting intermediate (3) and disaccharide hemiacetal form (8) were subjected to condensation via a Mitsunobu reaction, where the reaction proceeded in yield as high as 79% (only β) with complete β-selectivity. The protecting groups of the resulting compound were deprotected, thereby obtaining Novel steviol glycoside 1S (Reference compound 15).

Next, each of the synthesis steps will be described.

### (2) Synthesis of intermediate (3)

The intermediate (3) was synthesized according to the method as described in WO2018/181515.

### (3) Synthesis of disaccharide hemiacetal form

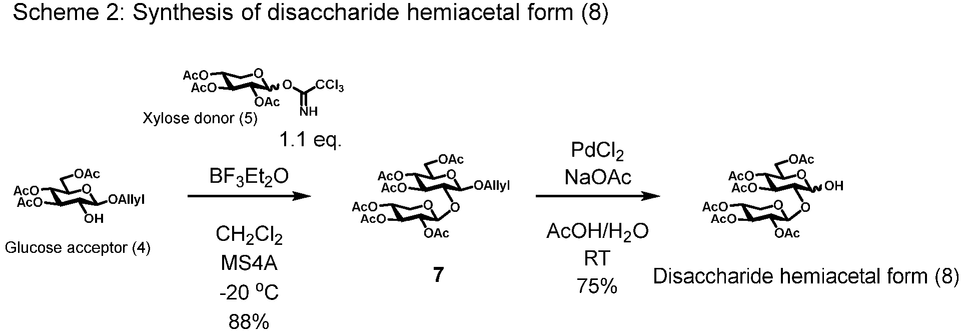

As can be appreciated from Scheme 2, for the synthesis of the disaccharide hemiacetal form (8), a glucose acceptor (4) (40.0 g, 115 mmol), a xylose donor (5) (53.4 g, 127 mmol) and 4Å molecular sieves (60 g) were dissolved in dichloromethane (1.2 L), to which a boron trifluoride-diethyl ether complex (1.46 mL, 11.5 mmol) was added at -20°C, and the resultant was agitated at -20°C for an hour. After confirming the completion of the reaction by TLC (ethyl acetate/hexane = 1/1, Rf value = 0.2), the resultant was neutralized with triethylamine (2.0 mL) (pH 8), and the 4Å molecular sieves were removed by filtration. The resultant was concentrated under a reduced pressure to obtain syrup, which was subjected to silica gel column chromatography to give Compound 7 (61.2 g, 88%) in the eluate (ethyl acetate/hexane = 1/1).

NMR spectra were determined for ¹H-NMR and ¹³C-NMR using "AVANCE III HD 400 spectrometer" manufactured by Bruker. The solvent and the frequencies used for the determinations were as follows. The same apparatus was used for determining the NMR spectra for other compounds described below.

### [Compound 7]

¹H-NMR (CDCl₃, 400 MHz) δ 2.01-2.10 (complex, 18H, OAc), 3.35 (dd, 1H), 3.68 (m, 1H), 3.73 (t, 1H), 4.13-4.27 (complex, 4H), 4.38 (m, 1H), 4.48 (d, J = 8.0 Hz, 1H), 4.74 (d, J = 6.4 Hz, 1H), 4.86 (t, 1H), 4.90-4.99 (complex, 2H), 5.09 (t, 1H), 5.35 (d, 1H), 5.91 (m, 1H); ¹³C-NMR (CDCl₃, 100 MHz) δ 20.6×2, 20.7×2, 20.8×2, 61.9, 62.0, 68.7, 68.9, 70.5, 70.7, 71.2, 71.5, 74.5, 98.9, 100.3, 117.8, 133.4, 169.4, 169.7, 170.0, 170.7.

Compound 7 (2.3 g, 3.8 mmol) was dissolved in acetic acid (100 mL) and water (10 mL), to which palladium chloride (1.2 g, 6.8 mmol) was added at room temperature, and the resultant was agitated in an argon atmosphere at room temperature for 18 hours. After confirming the completion of the reaction by TLC (chloroform/ethyl acetate = 2/1, Rf value = 0.2), palladium chloride was removed by filtration. The resultant was concentrated under a reduced pressure to obtain syrup, which was subjected to silica gel column chromatography to give the disaccharide hemiacetal form (8) (1.6 g, 75%) in the eluate (chloroform/ethyl acetate = 2/1).

### [Disaccharide hemiacetal form (8)]

¹H-NMR (CDCl₃, 400 MHz) δ 2.00-2.15 (complex, 27H, OAc), 3.31-3.39 (complex, 2H), 3.53-3.79 (complex, 3H), 3.86 (d, J = 2.8 Hz, 1H), 4.06-4.33 (complex, 6H), 4.58 (d, J = 7.2 Hz, 1H), 4.83-5.06 (complex, 5H), 5.09-5.22 (complex, 2H), 5.35 (m, 1H), 5.45 (t, 1H); ¹³C-NMR (CDCl₃, 100 MHz) δ 20.4, 20.5, 20.6×2, 20.7×2, 61.9, 62.2, 67.2, 68.3×2, 68.5, 68.7, 70.7, 71.4, 71.5, 76.7, 92.0, 101.6, 169.5, 169.7, 169.8×2, 170.1, 170.7.

### (4) Synthesis of Reference Compound 15

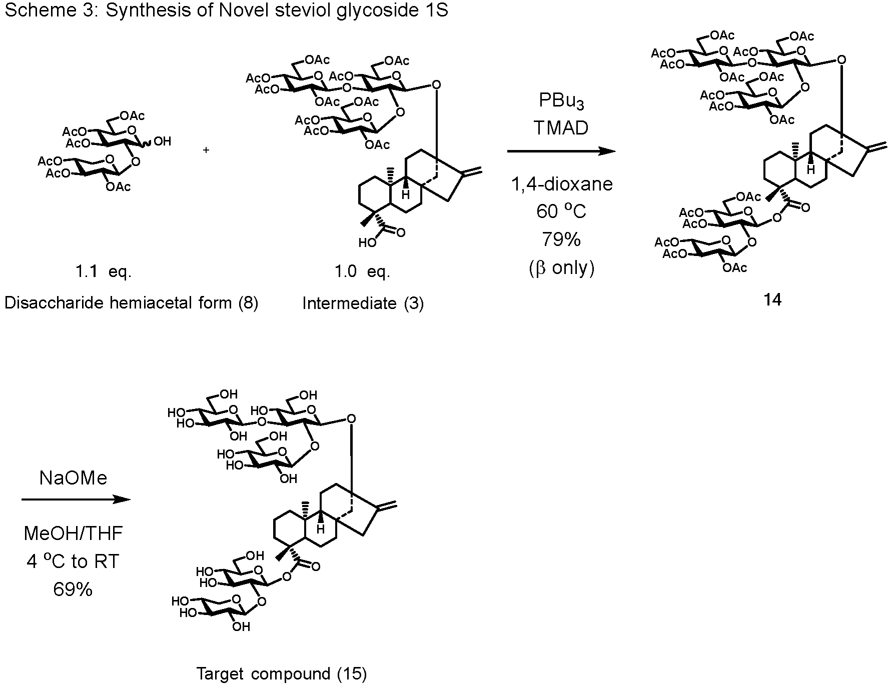

As can be appreciated from Scheme 3, for the synthesis of Compound 14, the disaccharide hemiacetal form (8) (9.4 g, 16.7 mmol) and the intermediate (3) (18.6 g, 15.1 mmol) were dissolved in 1,4-dioxane (150 mL), to which tributylphosphine (14.9 mL, 60.6 mmol) and 1,1'-azobis(N,N'-dimethylformamide) (TMAD) (10.4 g, 60.6 mmol) were added at room temperature and the resultant was agitated at 60°C for an hour. After confirming the completion of the reaction by TLC (toluene/ethyl acetate = 3/2, Rf value = 0.2), the resultant was diluted with ethyl acetate. The organic layer was washed with water, a saturated aqueous solution of sodium hydrogen carbonate and saturated saline, and dried with magnesium sulfate. Magnesium sulfate was removed by filtration. The resultant was concentrated under a reduced pressure to obtain syrup, which was subjected to silica gel column chromatography to give Compound 14 (21.1 g, 79%) in the eluate (toluene/ethyl acetate = 3/2).

### [Compound 14]

¹H-NMR (CDCl₃, 400 MHz) δ 0.75-1.32 (complex, 13H), 1.37-2.32 (complex, 77H), 3.36 (t, 1H), 3.50-3.72 (complex, 4H), 3.75-4.29 (complex, 14H), 4.41 (m, 1H), 4.52-4.65 (complex, 2H), 4.77-5.27 (complex, 20H), 5.62 (d, J = 7.6 Hz, 1H); ¹³C-NMR (CDCl₃, 100 MHz) δ 16.3, 19.3, 20.3, 20.4, 20.5×2, 20.6×3, 20.7×3, 20.9, 21.2, 21.3, 28.9, 36.5, 36.8, 39.3, 40.3, 41.1, 42.6, 43.3, 43.8, 45.1, 47.2, 53.5, 57.3, 60.3, 61.1, 61.7, 62.0, 62.6, 67.9, 68.0, 68.1, 68.3, 68.7, 70.6, 71.4, 71.6, 71.7, 71.8, 72.1, 72.9, 73.0, 74.7, 80.1, 85.8, 85.9, 91.2, 96.2, 98.9, 99.2, 100.9, 104.5, 125.2, 128.1, 128.7, 152.7, 168.9, 169.2, 169.3×2, 169.6, 169.7, 169.9, 170.0×2, 170.3×2, 170.5, 170.7, 174.5.

Compound (14) (20.0 g, 11.3 mmol) was dissolved in methanol (100 mL) and THF (100 mL), to which sodium methoxide (0.5M in methanol) (25 mL) was added at 4°C and the resultant was agitated at room temperature for an hour. After confirming the completion of the reaction by TLC (chloroform/methanol/water = 5/4/0.1, Rf value = 0.1), the resultant was neutralized by adding Amberlite 120B (H). The resultant was concentrated under a reduced pressure to obtain syrup, which was subjected to gel filtration column (GE Healthcare, Sephadex LH-20, ethanol) to give Reference Compound 15 (10.2 g, 69%).

### [Reference Compound 15]

¹H-NMR (pyridine-d5, 400 MHz) δ 0.70 (m, 1H), 0.88 (m, 1H), 0.91-1.40 (complex, 10H), 1.51-2.21 (complex, 14H), 2.43-2.55 (complex, 2H), 3.59-3.70 (complex, 2H), 3.77-4.51 (complex, 36H), 4.91 (s, 1H), 5.03 (d, J = 8.0 Hz, 1H), 5.20 (d, J = 7.6 Hz, 1H), 5.35 (d, J = 7.6 Hz, 1H), 5.52 (d, J = 8.0 Hz, 1H), 5.61 (m, 1H), 6.16 (d, J = 7.6 Hz, 1H); ¹³C-NMR (pyridine-d5, 100 MHz) δ 17.1, 20.4, 21.0, 22.9, 29.4, 38.1, 38.6, 40.2, 41.1, 42.3, 42.6, 44.4, 44.8, 45.9, 48.2, 54.5, 57.9, 62.6, 62.8×2, 63.8, 67.9, 70.4, 71.3, 71.6, 72.1, 72.8, 75.9, 76.3, 76.9, 77.9, 78.5, 78.7, 78.8, 79.0, 79.1, 79.5, 81.5, 82.3, 87.3, 88.6, 94.1, 98.2, 105.0, 105.1, 105.3, 107.1, 154.4, 176.4.

### [Synthesis of Novel steviol glycoside 2S]

### (1) Outline of synthetic pathways

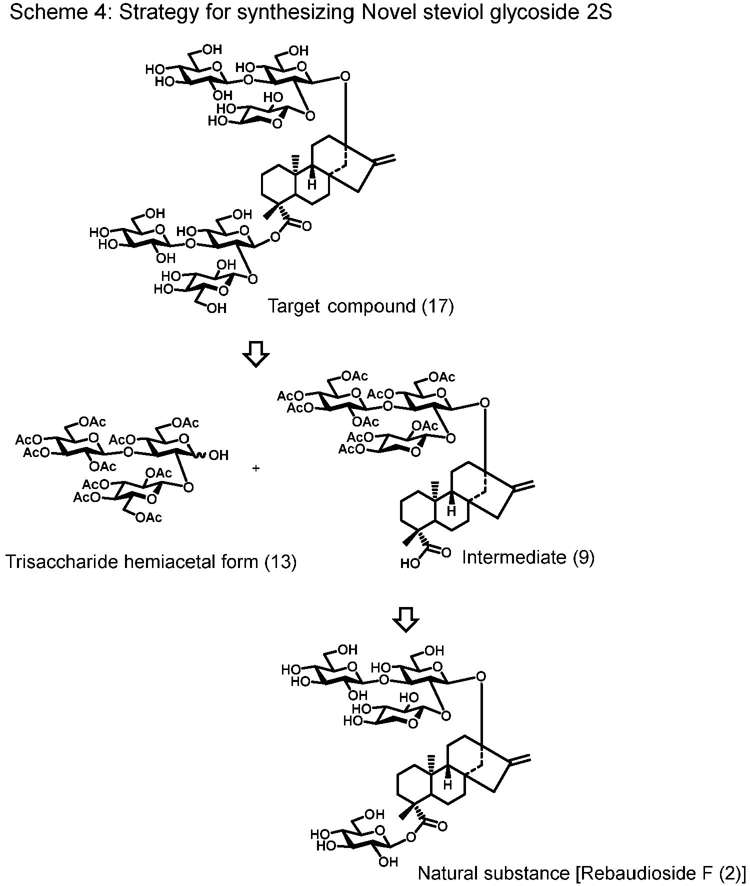

As can be appreciated from Scheme 4, for the synthesis of Novel steviol glycoside 2S (17), the intermediate (9) and the trisaccharide hemiacetal form (13) were condensed via a Mitsunobu reaction to obtain the backbone of Novel steviol glycoside 2S (17). For the synthesis of the intermediate (9), the ester bond at C-19 of steviol of the known natural substance, i.e., rebaudioside F (2), was subjected to alkaline hydrolysis and then the hydroxy groups of the sugar chain were protected with acetyl (Ac) groups to obtain the intermediate (9). The resulting intermediate (9) and the trisaccharide hemiacetal form (13) were subjected to condensation via a Mitsunobu reaction, where the reaction proceeded in yield as high as 53% (only β) with complete β-selectivity. The protecting groups of the resulting compound were deprotected, thereby obtaining Novel steviol glycoside 2S (17).

Next, each of the synthesis steps will be described.

### (2) Synthesis of intermediate (9)

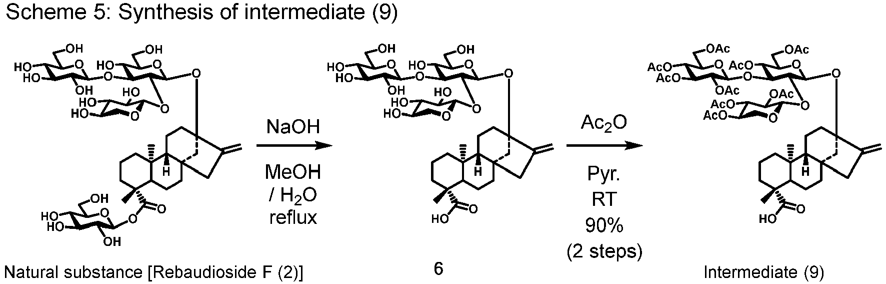

As can be appreciated from Scheme 5, for the synthesis of the intermediate (9), rebaudioside F (2) (1.8 g, 1.9 mmol) was dissolved in methanol (20 mL) and water (10 mL), to which 2 mol/L sodium hydroxide (10 mL) was added at room temperature, and the resultant was refluxed at 100°C for 3 hours. After confirming the completion of the reaction by TLC (chloroform/methanol = 4/1, Rf value = 0.2), the reaction solution was neutralized with Amberlite 120B (H) (pH 7). After the resin was removed by filtration, the resultant was concentrated under a reduced pressure to give Compound 6 (2.1 g)

Compound 6 (2.1 g) was dissolved in pyridine (20 mL), to which acetic anhydride (3.6 mL) was added at room temperature, and the resultant was agitated at room temperature for 24 hours. After confirming the completion of the reaction by TLC (chloroform/methanol = 50/1, Rf value = 0.2), a saturated aqueous solution of sodium hydrogen carbonate (40 mL) was added at 0°C and extraction was repeated for three times with ethyl acetate. The organic layer was concentrated under a reduced pressure to obtain syrup, which was subjected to silica gel column chromatography to give the intermediate 9 (2.0 g, 90% (2 steps)) in the eluate (chloroform/methanol = 50/1).

### [Intermediate 9]

¹H-NMR (CDCl₃, 400 MHz) δ 0.81 (m, 1H), 0.89-1.12 (complex, 8H), 1.22 (s, 3H), 1.41-2.22 (complex, 50H), 3.49 (dd, 1H), 3.58 (m, 1H), 3.65 (m, 1H), 3.85 (t, 1H), 3.96-4.15 (complex, 4H), 4.42 (m, 2H), 4.56 (d, J = 7.6 Hz, 1H), 4.81-4.94 (complex, 6H), 5.00 (t, 1H), 5.04-5.14 (complex, 3H), 5.25 (t, 1H); ¹³C-NMR (CDCl₃, 100 MHz) δ 16.0, 17.3, 19.1, 20.5, 20.6, 20.7, 20.8×2, 20.9, 21.8, 29.1, 29.8, 37.9, 38.0, 39.5, 40.7, 41.5, 42.2, 43.8, 44.0, 48.4, 53.8, 56.8, 61.7, 63.1, 66.8, 68.0, 68.7, 68.8, 69.7, 71.0, 71.6, 71.9, 72.4, 72.8, 81.3, 87.3, 96.6, 96.8, 99.2, 105.6, 151.9, 169.0, 169.5, 169.6, 170.1×2, 170.3, 170.6, 170.9, 171.0, 182.5.

### (3) Synthesis of trisaccharide hemiacetal form

The trisaccharide hemiacetal form was synthesized according to the method as described in WO2018/181515.

### (4) Synthesis of Compound 17

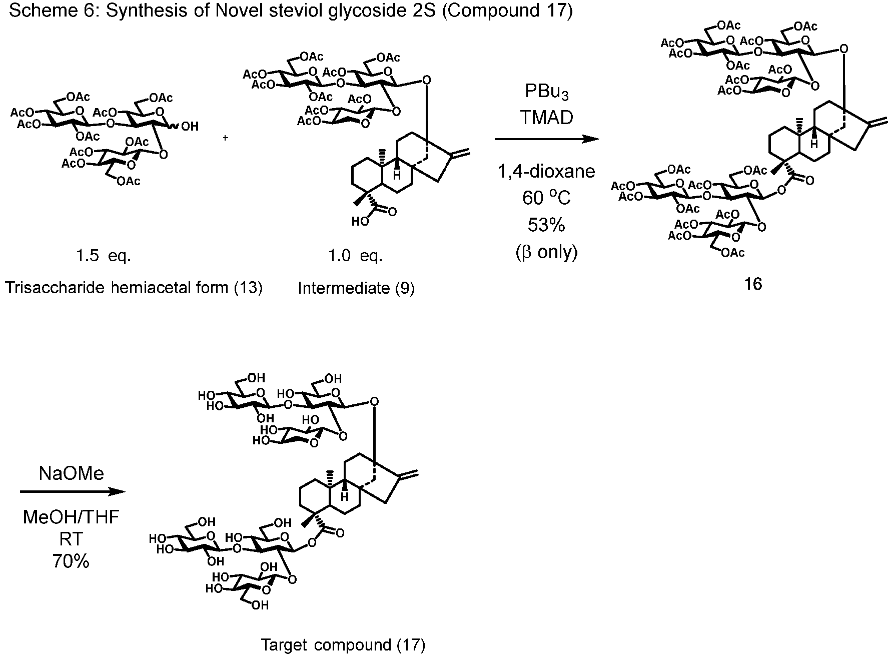

As can be appreciated from Scheme 6, for the synthesis of Compound 16, the trisaccharide hemiacetal form (13) (2.4 g, 2.6 mmol) and the intermediate (9) (2.0 g, 1.7 mmol) were dissolved in 1,4-dioxane (20 mL), to which tributylphosphine (4.3 mL, 17.3 mmol) and 1,1'-azobis(N,N'-dimethylformamide) (TMAD) (3.0 g, 17.3 mmol) were added at room temperature and the resultant was agitated at 60°C for 2 hours. After confirming the completion of the reaction by TLC (ethyl acetate/heptane = 2/1, Rf value = 0.1), the resultant was diluted with ethyl acetate. The organic layer was washed with water, a saturated aqueous solution of sodium hydrogen carbonate and saturated saline, and dried with magnesium sulfate. Magnesium sulfate was removed by filtration. The resultant was concentrated under a reduced pressure to obtain syrup, which was subjected to silica gel column chromatography to give Compound 16 (1.9 g, 53%, only β) in the eluate (ethyl acetate/heptane = 2/1).

### [Compound 16]

¹H-NMR (CDCl₃, 400 MHz) δ 0.78-1.05 (complex, 9H), 1.25 (t, 4H), 1.36-2.31 (complex, 86H), 3.51 (dd, 1H), 3.59 (m, 1H), 3.61-3.78 (complex, 4H), 3.81 (t, 1H), 3.91-4.21 (complex, 11H), 4.31 (dd, 1H), 4.40-4.59 (complex, 4H), 4.73-5.29 (complex, 21H), 5.60 (d, J = 7.2 Hz, 1H); ¹³C-NMR (CDCl₃, 100 MHz) δ 16.8, 17.5, 19.5, 20.5, 20.7×3, 20.8×3, 20.9×3, 21.0, 21.1×2, 21.5, 29.2, 37.4, 37.5, 39.6, 40.5, 41.6, 42.5, 43.8, 44.2, 48.1, 53.8, 57.4, 61.7, 62.0, 62.1, 62.3, 63.1, 66.7, 67.4, 68.0, 68.3, 68.4, 68.6, 68.8, 69.7, 71.1, 71.5, 71.8, 71.9, 72.0, 72.2, 72.3, 72.4, 72.9, 73.1, 75.0, 80.1, 81.5, 86.8, 91.3, 96.4, 97.0, 99.2, 99.3, 99.6, 104.9, 152.8, 169.0, 169.1, 169.3, 169.5×2, 169.6, 170.1×2, 170.2×3, 170.5, 170.6, 170.9, 174.8.

Compound (16) (2.2 g, 1.1 mmol) was dissolved in methanol (10 mL) and THF (10 mL), to which sodium methoxide (0.5M in MeOH) (2.5 mL) was added at room temperature, and the resultant was agitated at room temperature for 3 hours. After confirming the completion of the reaction by TLC (chloroform/methanol/water = 5/4/1, Rf value = 0.4), the resultant was neutralized with Amberlite 120B (H) (pH 7). After the resin was removed by filtration, the resultant was concentrated under a reduced pressure, which was dissolved in MeCN/H₂O = 1/2 and lyophilized to give Compound 17 (1.0 g, 70%).

### [Compound 17]

¹H-NMR (pyridine-d5, 400 MHz) δ 0.78 (m, 1H), 0.91 (m, 1H), 1.08 (m, 1H), 1.31-1.48 (complex, 9H), 1.62-1.80 (complex, 3H), 1.81-1.89 (complex, 3H), 2.00-2.06 (complex, 2H), 2.29 (m, 3H), 2.47 (m, 1H), 2.69 (m, 1H), 2.80 (m, 1H), 3.41 (t, 1H), 3.81 (m, 1H), 3.90-4.41 (complex, 33H), 4.53-4.61 (complex, 2H), 4.70 (m, 1H), 4.88-5.22 (complex, 14H), 5.30 (d, J = 8.0 Hz, 1H), 5.37 (d, J = 7.6 Hz, 1H), 5.51-5.57 (complex, 2H), 5.61 (s, 1H), 5.77 (s, 2H), 5.84 (d, J = 7.6 Hz, 1H), 6.46 (d, J = 8.0 Hz, 1H); ¹³C-NMR (pyridine-d5, 100 MHz) δ 18.3, 21.2, 21.7, 25.1, 29.8, 39.9, 41.3, 41.7, 42.5, 44.2, 44.7, 45.8, 47.9, 55.9, 58.9, 63.3, 63.5, 63.6, 64.2, 65.6, 68.5, 71.7, 72.2, 72.6, 72.9, 75.2, 76.9, 77.0, 77.2, 78.4, 79.2, 79.3, 79.4, 79.5, 79.6, 79.9, 80.0, 80.6, 83.7, 89.2, 89.5, 90.2, 96.5, 97.7, 105.4, 105.7, 105.9, 106.4, 107.3, 154.9, 178.5.
(iii) Structural determination by matching with the retention time and the fragmented pattern of the chemically synthesized standard product in HPLC-high resolution MS and MS³-fragmentation

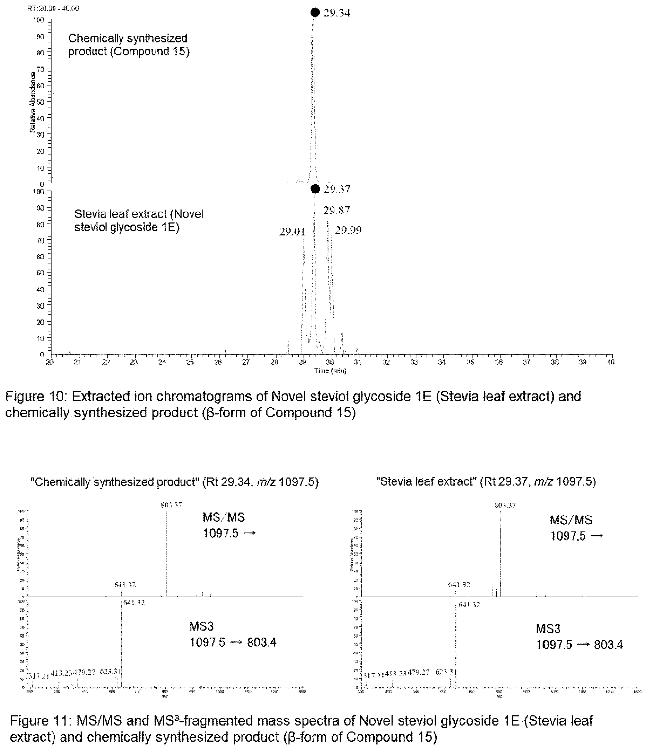

The chemically synthesized product of Novel steviol glycoside 1 (β-form of Reference Compound 15) and the stevia leaf liquid extract were compared by HPLC-high resolution MS/MS and MS³-fragmentation by using Orbitrap Elite MS (from Thermo Fisher Scientific) equipped with a HPLC-ESI ion source under the conditions described in (i). As a result, the peaks of the chemically synthesized product and the stevia leaf liquid extract were detected at the retention time of 29.34 and 29.37 minutes, respectively (Figure 10). Here, the peaks at the retention time of 29.34 and 29.37 minutes in Figure 10 corresponded to the peak at the retention time of 29.05 minutes in Figure 2, which was confirmed by determining the retention time of the chemically synthesized product with the apparatus used in Figure 2 (LCMS-8030). Moreover, they also matched in the respective MS/MS and MS³-fragmented mass spectra (Figure 11). From this result, Novel steviol glycoside 1E obtained from the liquid extract of the plant was confirmed to have the same structure as the β-form of Reference Compound 15.

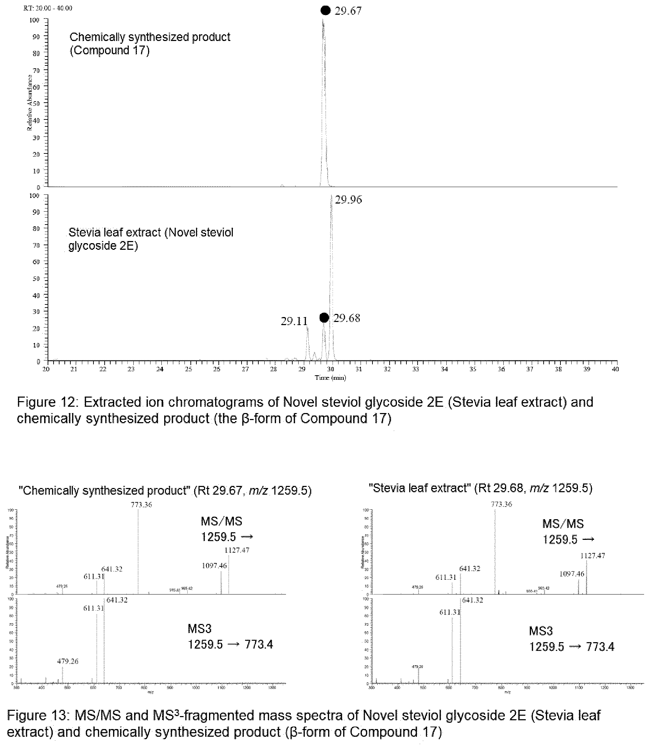

Furthermore, the chemically synthesized product of Novel steviol glycoside 2 (β-form of Compound 17) and the stevia leaf liquid extract were compared by HPLC-high resolution MS/MS and MS³-fragmentation by using Orbitrap Elite MS (from Thermo Fisher Scientific) equipped with a HPLC-ESI ion source under the conditions described in (i). As a result, the peaks of the chemically synthesized product and the stevia leaf liquid extract were detected at the retention time of 29.67 and 29.68 minutes, respectively (Figure 12). Here, the peaks at the retention time of 29.67 and 29.68 minutes in Figure 12 corresponded to the peak at the retention time of 29.30 minutes in Figure 3, which was confirmed by determining the retention time of the chemically synthesized product with the apparatus used in Figure 3 (LCMS-8030). Moreover, they also matched in the respective MS/MS and MS³-fragmented mass spectra (Figure 13). From this result, Novel steviol glycoside 2E obtained from the liquid extract of the plant was confirmed to have the same structure as the β-form of Compound 17.

### Evaluation of sweetness level of novel steviol glycosides

In order to evaluate the sweetness levels of Novel steviol glycosides A and B, samples were prepared by adding sugar to pure water to give Brix of 3.0 to 5.0 in 0.5 increments. Reference Compound 15 and Compound 17 obtained by the chemical syntheses were used as Reference steviol glycoside A and Novel steviol glycoside B, respectively, where 48 mg of each sample was dissolved in 400 mL of pure water to be tested. In addition, samples were also prepared for comparison by dissolving 48 mg of each of Reb.A, Reb.D and Reb.M in 400 mL of pure water.

### (1) Evaluation of sweetness level of Reference steviol glycoside A

Evaluation was conducted by choosing a sugar-added sample that had an equivalent sweetness intensity to that of the sample added with the reference steviol glycoside, where sensory evaluation was conducted by panelists who had been trained about sensory attributes of sweeteners (7 members). As a result, reference steviol glycoside A was found to have a sweetness level that was about 354 times higher in average than that of sugar.

**Table 2: Evaluation of sweetness level of Reference steviol glycoside A**

| Sweetness level | Reference steviol glycoside A | Reb A | Reb D | Reb M |
|---|---|---|---|---|
| Lowest | 319.0 | 243.0 | 253.5 | 286.0 |
| Highest | 415.8 | 398.9 | 398.0 | 352.2 |
| Average | 354.1 | 312.0 | 317.9 | 341.1 |

### (2) Evaluation of sweetness level of Novel steviol glycoside B

Sweetness level was evaluated in the same manner as reference steviol glycoside A. As a result, Novel steviol glycoside B of the present invention was found to have a sweetness level that was about 250 times higher in average than that of sugar. The results are shown in the table below.

**Table 3: Evaluation of sweetness level of Novel steviol glycoside B**

| Sweetness level | Novel steviol glycoside B | Reb A | Reb D | Reb M |
|---|---|---|---|---|
| Lowest | 223.6 | 239.5 | 241.1 | 236.5 |
| Highest | 299.2 | 329.2 | 393.6 | 373.4 |
| Average | 249.6 | 288.3 | 310.3 | 323.6 |

### Sensory evaluation of Reference steviol glycoside A (Reference Compound 15)

In order to evaluate the taste quality of various steviol glycosides, Reb.A, Reb.D, Reb.M, sugar and reference steviol glycoside A (Reference Compound 15) were each added to pure water to prepare beverage samples. All of the beverage samples were adjusted to have final sweetness level (Brix) of 5 in terms of sugar (sucrose), and thus the sweetness levels of Reb.A, Reb.D, Reb.M and reference steviol glycoside A (Reference Compound 15) were 312, 317, 341 and 354, respectively.

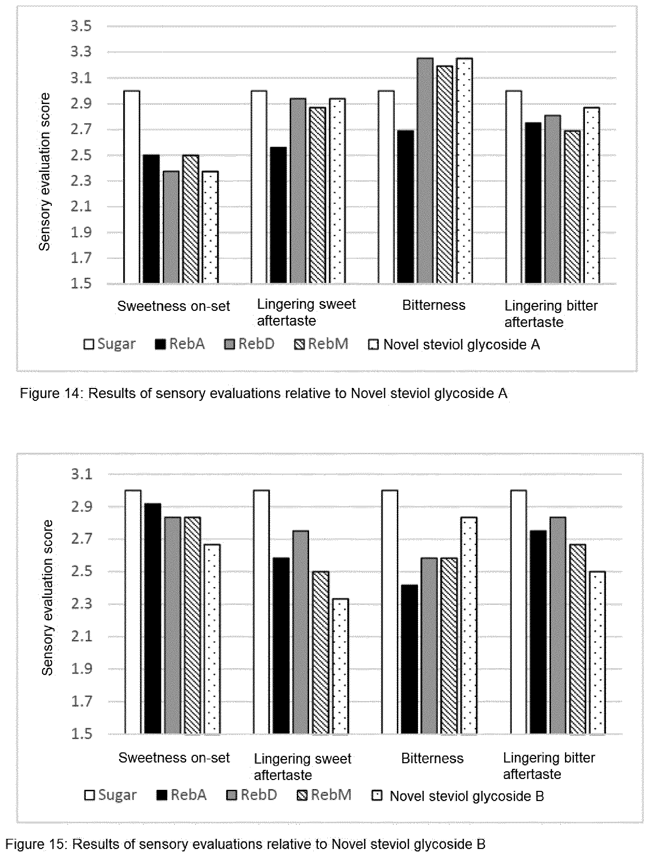

The resulting beverage samples were subjected to sensory evaluation for rating the attributes, which were sweetness on-set, lingering sweet aftertaste, bitterness and lingering bitter aftertaste. Panelists who had been trained about sensory attributes of sweeteners (7 members) evaluated based on the following evaluation criteria. For each evaluation item, the steviol glycosides were scored in 0.5 increments provided that the score of sugar was 3. A higher score represents faster sweetness on-set, shorter lingering sweet aftertaste, less bitterness and shorter lingering bitter aftertaste. The results are shown in Figure 14. The evaluation scores shown in the diagram are the average scores of the scores from the 8 panelists. As a result of the sensory evaluations, reference steviol glycoside A was found to have less sweetness and shorter lingering bitter aftertaste as compared to other glycosides and less bitterness as compared to other components including sugar.

### Sensory evaluation of Novel steviol glycoside B (Compound 17)

In order to evaluate the taste quality of various steviol glycosides, Reb.A, Reb.D, Reb.M, sugar and Novel steviol glycoside B (Compound 17) were each added to pure water to prepare beverage samples. All of the beverage samples were adjusted to have final sweetness level (Brix) of 5 in terms of sugar (sucrose), and thus the sweetness levels of Reb.A, Reb.D, Reb.M and Novel steviol glycoside B (Compound 17) were 288, 310, 324 and 250, respectively.

The resulting beverage samples were subjected to sensory evaluation for rating the attributes, which were sweetness on-set, lingering sweet aftertaste, bitterness and lingering bitter aftertaste. Panelists who had been trained about sensory attributes of sweeteners (7 members) evaluated based on the following evaluation criteria. For each evaluation item, the steviol glycosides were scored in 0.5 increments provided that the score of sugar was 3. A higher score represents faster sweetness on-set, shorter lingering sweet aftertaste, less bitterness and shorter lingering aftertaste. The results are shown in Figure 15. The evaluation scores shown in the diagram are the average scores of the scores from the 6 panelists. As a result of the sensory evaluations, Novel steviol glycoside B was found to have less bitterness as compared to other glycosides.

### Identification of genetic features of plant containing reference steviol glycosides A and Novel steviol glycoside B

Lines rich in reference steviol glycosides A and Novel steviol glycoside B (Mutants: Cultivar lines A and B) and lines with small reference steviol glycosides A and Novel steviol glycoside B contents (Wild-types: Cultivar lines X and Y) were used to identify the genetic features specific to the lines rich in reference steviol glycosides A and Novel steviol glycoside B. When the genomes of the respective lines were sequenced, the lines rich in reference steviol glycosides A and Novel steviol glycoside B were found to have substitutions at the 298th, 328th, 360th, 386th, 393rd, 411th, 427th and 453rd bases of SEQ ID NO:1, insertion of 15 bases between the 90th and 91st bases of SEQ ID NO:57, and substitutions at the 98th, 102nd, 111th, 113th, 116th, 119th and 122nd bases of SEQ ID NO:59 with respect to the wild-types (nucleotide sequences of the mutant lines corresponding to SEQ ID NOS:1, 57 and 59 are shown as SEQ ID NOS:2, 58 and 60, respectively). In addition, the substitutions in SEQ ID NO:1, the insertion in SEQ ID NO:57 and the substitutions in SEQ ID NO:59 were found to exist in the introns of the genes coding for the protein including the amino acid sequences represented by SEQ ID NOS:53, 61 and 63, respectively (herein, sometimes referred to as P1, P2 and P3). Subsequently, whether or not these mutations affect the expression level of each gene was examined. After 100 mg of the expanded leaves of Cultivars A, B, X (SR001) and Y (SS075-49) were cryogenically ground with liquid nitrogen, total RNA was extracted using RNAeasy Plant mini kit from QIAGEN according to the manufacturer's protocol. 500 ng of the extracted total RNA was used for reverse transcription. For the reverse transcription, cDNA was synthesized using SuperScript IV VILO manufactured by Thermo Fisher Scientific according to the manufacturer's protocol. Semi-quantitative PCR was conducted using 1 µL of a 10-fold dilution of the reverse transcription reaction solution. Semi-quantitative PCR was carried out with Ampdirect manufactured by Shimadzu Corporation according to the manufacturer's protocol. PCR reaction was performed by heat denaturation at 95°C for 10 minutes, followed by 32, 33 and 28 cycles of reactions at 94°C for 30 seconds, 55°C for 30 seconds and 72°C for 25 seconds, for "P3", "P1 and P2" and "actin and ubiquitin as controls", respectively. PCR reaction was followed by electrophoresis using LabChip GX Touch manufactured by PerkinElmer according to the manufacturer's protocol.

The following primers were used for semi-quantitative PCR.
P1
   Forward: CCTGTTCATTACAAATTCAACCCG (SEQ ID NO:55)
   Reverse: AACCCTAACATGTTCAATGTCCCTA (SEQ ID NO:56)
P2
   Forward: ATCAGATTTATCATCTTGCATGCCC (SEQ ID NO:65)
   Reverse: TGCCAATTACATTCGTCTTAATCGT (SEQ ID NO:66)
P3
   Forward: AAAAGTTGCTGGTTGAAGTTGATCA (SEQ ID NO:67)
   Reverse: CACACTAAATATGCTTGGTCTTGC (SEQ ID NO:68)
Actin
   Forward: CGCCATCCTCCGTCTTGATCTTGC (SEQ ID NO:69)
   Reverse: CCGTTCGGCGGTGGTGGTAA (SEQ ID NO:70)
Ubiquitin
   Forward: TCACTCTTGAAGTGGAGAGTTCCGA (SEQ ID NO:71)
   Reverse: GCCTCTGTTGGTCCGGTGGG (SEQ ID NO:72)

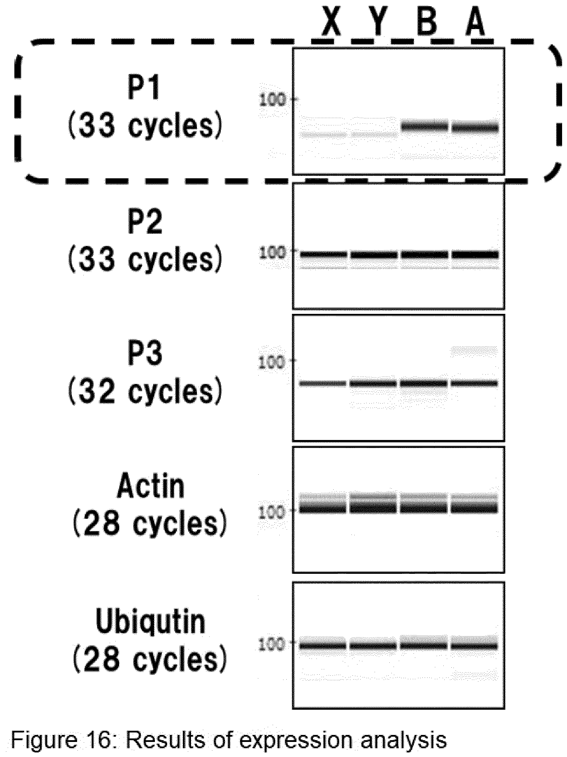

The results of the expression analysis are shown in Figure 16 and Table 4. The numerical values shown in Table 4 are the relative band intensities of the P1-P3 genes to the band intensity of the ubiquitin gene, i.e., 100, in the electrophoresis image shown in Figure 16.

**Table 4: Expression levels of P1-P3 genes in each cultivar**

| Sample name | P1 | P2 | P3 |
|---|---|---|---|
| Cultivar A | 146.27 | 126.23 | 99.01 |
| Cultivar B | 124.57 | 107.76 | 93.79 |
| Cultivar X | 0.20 | 72.14 | 40.23 |
| Cultivar Y | 0.13 | 94.66 | 77.95 |

From these results, the P1 gene was confirmed to be highly expressed in the two lines including reference steviol glycosides A and Novel steviol glycoside B. The mutations found in the P1 gene exist in the introns and the existence of one or more of these mutations seemed to enhance the expression level of P1, by which the syntheses of reference steviol glycosides A and Novel steviol glycoside B were promoted in the plant.

### Identification of content of Reference Glycoside A in plant having genetic features 1-8

Suitable amounts of fresh leaves were sampled from individuals of Cultivars A, B, X and Y that were used in the above-described "Identification of genetic features of plant containing reference steviol glycosides A and Novel steviol glycoside B " to quantify the concentrations of the sweetness components by LC/MS-MS (Shimadzu LCMS8050). Specifically, a prescribed amount of fresh leaves was freeze-dried, and the crushed dry pieces were fed into pure water. The resultant was subjected to an ultrasonic treatment for 20 minutes for extraction, centrifuged and filtrated to give 5 mL of a liquid extract. This liquid extract was subjected to LC/MS-MS analysis with LCMS8050 in ion mode (Shimadzu LCMS8050) to quantify the concentrations of Reb.A, Reb.B, Reb.C, Reb.D, Reb.E, Reb.F, Reb.G, Reb.I, Reb.M, Reb.N, stevioside, dulcoside A, steviol bioside, rubusoside and Novel steviol glycoside A. The total thereof was considered to be the concentration of the sweetness components (amount of total steviol glycosides (TSG) of this example). All of the water content of the dried leaves was less than about 3%. The results are shown in Tables 5-9. The values of the following results are the average values of two measurements.

**Table 5: Amount of TSG in dried leaves of each cultivar**

| **Sample name** | **Weight of stevia leaves (g)** | **Concentration of TSG (mg/L)** | **Amount of TSG (mg)** | **Rate of TSG (%)** |
|---|---|---|---|---|
| **Cultivar A** | 0.0565 | 1617.35 | 8.087 | 14.3% |
| **Cultivar B** | 0.0501 | 905.42 | 4.527 | 9.0% |
| **Cultivar X** | 0.0445 | 1142.40 | 5.712 | 12.9% |
| **Cultivar Y** | 0.0576 | 1267.14 | 6.336 | 11.1% |

**Table 6: Rate of each steviol glycoside in TSG contained in dried leaves of each cultivar**

| | **Rate of each steviol glycoside in TSG** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside A** | **Total*** |
| **Cultivar A** | 40.4% | 0.2% | 36.6% | 0.7% | 11.0% | 7.9% | 0.2% | 0.2% | 0.061% | 100% |
| **Cultivar B** | 64.6% | 0.6% | 11.8% | 7.7% | 5.5% | 4.0% | 2.3% | 1.3% | 0.205% | 100% |
| **Cultivar X** | 24.1% | 0.1% | 6.1% | 0.5% | 62.4% | 1.2% | 0.0% | 0.1% | 0.017% | 100% |
| **Cultivar Y** | 38.9% | 0.2% | 6.5% | 1.9% | 47.9% | 1.7% | 0.1% | 0.4% | 0.045% | 100% |

| | | | | | | | | | | |
|---|---|---|---|---|---|---|---|---|---|---|
| *: Total content includes Reb A, Reb B, Reb C, Reb D, Reb E, Reb F, Reb G, Reb I, Reb M, Reb N, stevioside, dulcoside A, steviol bioside, rubusoside and reference steviol glycoside A. | | | | | | | | | | |

**Table 7: Rate of reference glycoside A relative to eight types of major steviol glycosides^{*1}**

| Sample name | Rate of steviol glycoside A |
|---|---|
| Cultivar A | 0.063% |
| Cultivar B | 0.210% |
| Cultivar X | 0.018% |
| Cultivar Y | 0.046% |

| | |
|---|---|
| ^{*1}: Reb A, Reb B, Reb C, Reb D, Reb F, Reb M, Reb N and stevioside recited in Table 6 | |

**Table 8: Rate of each steviol glycoside contained in dried leaves of each cultivars**

| | **Content of each steviol glycoside in dried leaves** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside A** | **TSG** |
| **Cultivar A** | 5.8% | 0.0% | 5.2% | 0.1% | 1.6% | 1.1% | 0.0% | 0.0% | 0.009% | 14.3% |
| **Cultivar B** | 5.8% | 0.1% | 1.1% | 0.7% | 0.5% | 0.4% | 0.2% | 0.1% | 0.019% | 9.0% |
| **Cultivar X** | 3.1% | 0.0% | 0.8% | 0.1% | 8.0% | 0.1% | 0.0% | 0.0% | 0.002% | 12.9% |
| **Cultivar Y** | 4.3% | 0.0% | 0.7% | 0.2% | 5.3% | 0.2% | 0.0% | 0.0% | 0.005% | 11.1% |

**Table 9: Rate of reference steviol glycoside A relative to each steviol glycoside contained in dried leaves of each cultivar**

| | **Rate of reference steviol glycoside A relative to each steviol glycoside contained in dried leaves** | | | | | | | | |
|---|---|---|---|---|---|---|---|---|---|
| **Sample name** | **Reb A** | **Reb B** | **Reb C** | **Reb D** | **Stevioside** | **Reb F** | **Reb M** | **Reb N** | **Reb A+ Stevioside** |
| **Cultivar A** | 0.15% | 30.00% | 0.16% | 8.57% | 0.55% | 0.76% | 30.00% | 30.00% | 0.12% |
| **Cultivar B** | 0.33% | 35.00% | 1.78% | 2.73% | 3.82% | 5.25% | 9.13% | 16.15% | 0.30% |
| **Cultivar X** | 0.08% | 20.00% | 0.33% | 4.00% | 0.03% | 1.67% | - | 20.00% | 0.02% |
| **Cultivar Y** | 0.13% | 25.00% | 0.77% | 2.63% | 0.10% | 2.94% | 50.00% | 12.50% | 0.06% |

### Identification of content of Novel Glycoside B in plant having genetic features 1-8

Suitable amounts of fresh leaves were sampled from individuals of Cultivars B and X that were used in the above-described "Identification of genetic features of plant containing reference steviol glycosides A and Novel steviol glycoside B " to quantify the concentrations of the sweetness components by LC/MS-MS (Shimadzu LCMS8050). Specifically, a prescribed amount of fresh leaves was freeze-dried, and the crushed dry pieces were fed into pure water. The resultant was subjected to an ultrasonic treatment for 20 minutes for extraction, centrifuged and filtrated to give 5 mL of a liquid extract. This liquid extract was subjected to LC/MS-MS analysis with LCMS8050 in ion mode (Shimadzu LCMS8050) to quantify the concentrations of Reb.A, Reb.B, Reb.C, Reb.D, Reb.E, Reb.F, Reb.G, Reb.I, Reb.M, Reb.N, stevioside, dulcoside A, steviol bioside, rubusoside and Novel steviol glycoside B. The total thereof was considered to be the concentration of the sweetness components (amount of total steviol glycosides (TSG) of this example). All of the water content of the dried leaves was less than about 3%. The results are shown in Tables 10-14.

**Table 10: Amount of TSG in dried leaves of each cultivar**

| **Sample name** | **Weight of stevia leaves (g)** | **Concentration of TSG (mg/L)** | **Amount of TSG (mg)** | **Rate of TSG (%)** |
|---|---|---|---|---|
| **Cultivar B** | 0.0524 | 777.42 | 3.887 | 7.4% |
| **Cultivar X** | 0.0553 | 1672.45 | 8.362 | 15.1% |

**Table 11: Rate of each steviol glycoside in TSG contained in dried leaves of each cultivar**

| | **Rate of each steviol glycoside in TSG** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside B** | **Total*** |
| **Cultivar B** | 66.6% | 0.7% | 7.2% | 10.0% | 4.1% | 2.2% | 4.9% | 1.9% | 1.328% | 100% |
| **Cultivar X** | 30.8% | 0.1% | 6.0% | 0.8% | 58.0% | 1.1% | 0.0% | 0.1% | 0.000% | 100% |

| | | | | | | | | | | |
|---|---|---|---|---|---|---|---|---|---|---|
| *: Total content includes Reb A, Reb B, Reb C, Reb D, Reb E, Reb F, Reb G, Reb I, Reb M, Reb N, stevioside, dulcoside A, steviol bioside, rubusoside and Novel steviol glycoside B. | | | | | | | | | | |

**Table 12: Rate of Novel glycoside B relative to eight types of major steviol glycosides^{*1}**

| Sample name | Rate of steviol glycoside B |
|---|---|
| Cultivar B | 1.360% |
| Cultivar X | 0.000% |

| | |
|---|---|
| *¹: Reb A, Reb B, Reb C, Reb D, Reb F, Reb M, Reb N and stevioside recited in Table 11 | |

**Table 13: Rate of each steviol glycoside contained in dried leaves of each cultivars**

| | **Content of each steviol glycoside in dried leaves** | | | | | | | | | |
|---|---|---|---|---|---|---|---|---|---|---|
| **Sample name** | **Reb A** | **Reb B** | **Reb C** | **Reb D** | **Stevioside** | **Reb F** | **Reb M** | **Reb N** | **Novel steviol glycoside B** | **TSG** |
| **Cultivar B** | 4.9% | 0.1% | 0.5% | 0.7% | 0.3% | 0.2% | 0.4% | 0.1% | 0.099% | 7.4% |
| **Cultivar X** | 4.7% | 0.0% | 0.9% | 0.1% | 8.8% | 0.2% | 0.0% | 0.0% | 0.000% | 15.1% |

**Table 14: Rate of Novel steviol glycoside B relative to each steviol glycoside contained in dried leaves of each cultivar**

| | **Rate of Novel steviol glycoside B relative to each steviol glycoside contained in dried leaves** | | | | | | | | |
|---|---|---|---|---|---|---|---|---|---|
| **Sample name** | **Reb A** | **Reb B** | **Reb C** | **Reb D** | **Stevioside** | **Reb F** | **Reb M** | **Reb N** | **Reb A+ Stevioside** |
| **Cultivar B** | 1.99% | 178.85% | 18.32% | 13.29% | 32.22% | 59.12% | 27.25% | 71.02% | 1.88% |

## Claims

1. A compound of Formula (3) or a salt or a hydrate thereof :

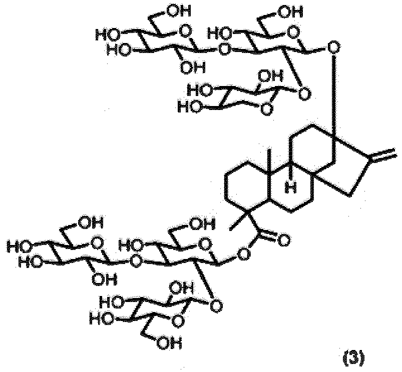

2. The compound according to Claim 1, or a salt or a hydrate thereof, wherein the compound is represented by Formula (5) below:

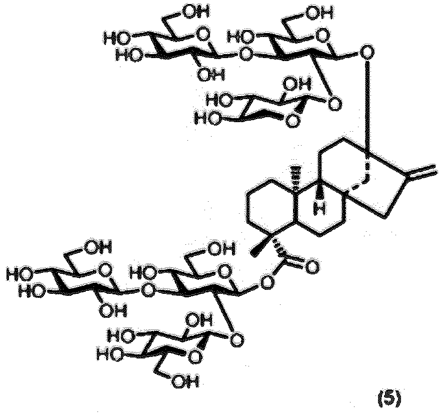

3. A food or beverage comprising the compound according to Claim 1 or 2.

4. The food or beverage according to Claim 3, wherein the content of the compound according to Claim 1 or 2 is 1-600 mass ppm.

5. A sweetener composition comprising the compound according to Claim 1 or 2.

6. The sweetener composition according to Claim 5, wherein the content of the compound according to Claim 1 or 2 is 1-99 wt%.

7. The sweetener composition according to Claim 5 or 6, further comprising one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, dulcoside C, rubusoside, steviol, steviol monoside, steviol bioside and stevioside.

8. A food or beverage comprising the sweetener composition according to any one of Claims 5 to 7.

9. The food or beverage according to Claim 8, which is a beverage.

10. Use of the compound according to Claim 1 or 2 as a sweetener.

## Patentansprüche

1. Verbindung der Formel (3) oder ein Salz oder ein Hydrat davon:

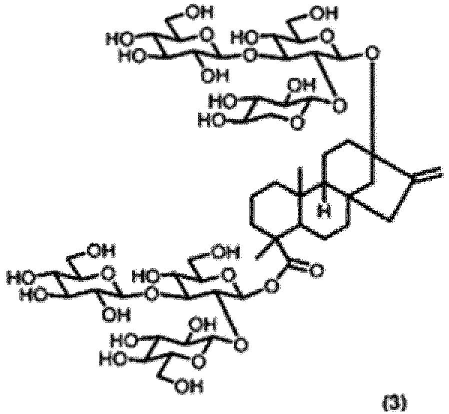

2. Verbindung nach Anspruch 1 oder ein Salz oder ein Hydrat davon, wobei die Verbindung durch die nachstehende Formel (5) dargestellt ist:

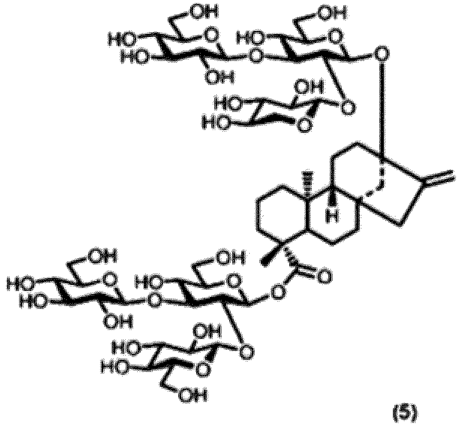

3. Lebensmittel oder Getränk, das eine Verbindung nach Anspruch 1 oder 2 umfasst.

4. Lebensmittel oder Getränk nach Anspruch 3, wobei der Gehalt der Verbindung nach Anspruch 1 oder 2 1 bis 600 Massen-ppm beträgt.

5. Süßstoffzusammensetzung, die eine Verbindung nach Anspruch 1 oder 2 umfasst.

6. Süßstoffzusammensetzung nach Anspruch 5, wobei der Gehalt der Verbindung nach Anspruch 1 oder 2 1 bis 99 Gew.-% beträgt.

7. Süßstoffzusammensetzung nach Anspruch 5 oder 6, die weiters eine oder mehrere Arten von Steviolglykosiden, ausgewählt aus der aus Rebaudiosid A, Rebaudiosid B, Rebaudiosid C, Rebaudiosid D, Rebaudiosid E, Rebaudiosid F, Rebaudiosid I, Rebaudiosid J, Rebaudiosid K, Rebaudiosid N, Rebaudiosid M, Rebaudiosid O, Rebaudiosid Q, Rebaudiosid R, Dulcosid A, Dulcosid C, Rubusosid, Steviol, Steviolmonosid, Steviolbiosid und Steviosid bestehenden Gruppe umfasst.

8. Lebensmittel oder Getränk, das eine Süßstoffzusammensetzung nach einem der Ansprüche 5 bis 7 umfasst.

9. Lebensmittel oder Getränk nach Anspruch 8, das ein Getränk ist.

10. Verwendung einer Verbindung nach Anspruch 1 oder 2 als Süßstoff.

## Revendications

1. Composé de formule (3), ou un sel ou un hydrate de celui-ci :

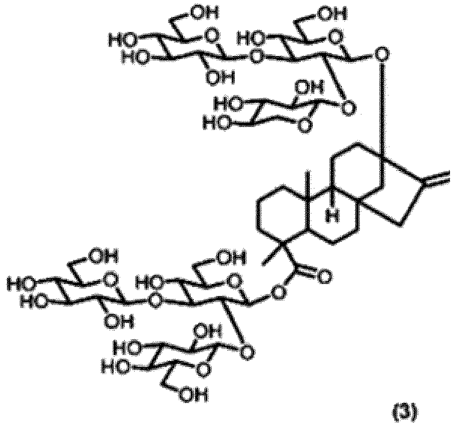

2. Composé selon la revendication 1, ou un sel ou un hydrate de celui-ci, dans lequel le composé est représenté par la formule (5) ci-dessous :

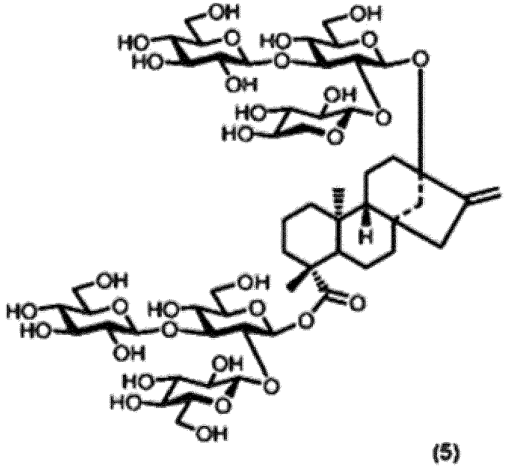

3. Aliment ou boisson comprenant le composé selon la revendication 1 ou 2.

4. Aliment ou boisson selon la revendication 3, dans lequel la teneur du composé selon la revendication 1 ou 2 est de 1 à 600 ppm en masse.

5. Composition d'édulcorant comprenant le composé selon la revendication 1 ou 2.

6. Composition d'édulcorant selon la revendication 5, dans laquelle la teneur du composé selon la revendication 1 ou 2 est de 1 à 99 % en poids.

7. Composition d'édulcorant selon la revendication 5 ou 6, comprenant en outre un ou plusieurs types de glycosides de stéviol choisis dans le groupe comprenant rébaudioside A, rébaudioside B, rébaudioside C, rébaudioside D, rébaudioside E, rébaudioside F, rébaudioside I, rébaudioside J, rébaudioside K, rébaudioside N, rébaudioside M, rébaudioside O, rébaudioside Q, rébaudioside R, dulcoside A, dulcoside C, rubusoside, stéviol, monoside de stéviol, bioside de stéviol et stévioside.

8. Aliment ou boisson comprenant la composition d'édulcorant selon l'une quelconque des revendications 5 à 7.

9. Aliment ou boisson selon la revendication 8, qui est une boisson.

10. Utilisation du composé selon la revendication 1 ou 2 comme édulcorant.
